# EUROPEAN PATENT SPECIFICATION

(11) **EP 1 503 780 B1**
(45) Date of publication and mention of the grant of the patent: **26.08.2009**
(21) Application number: 03724331.8
(22) Date of filing: 09.05.2003
(51) Int. Cl.: A61K 38/00, A61K 48/00, C12Q 1/68, G01N 33/53, G01N 33/543, C07K 14/00

(54) **ANDROGEN-REGULATED PMEPA1 GENE AND METHODS OF USING THE SAME TO INHIBIT CANCER CELL GROWTH**
ANDROGEN-REGULIERTES PMEPA1 GEN UND VERFAHREN ZU SEINER VERWENDUNG ZUR HEMMUNG VON KREBSZELLWACHSTUM
GENE PMEPA1 REGULE PAR LES ANDROGENES ET PROCEDES D'UTILISATION DE CE DERNIER POUR INHIBER LA CROISSANCE DES CELLULES CANCEREUSES

(30) Priority: 10.05.2002 US 378949 P
(43) Date of publication of application: 09.02.2005
(73) Proprietor: HENRY M. JACKSON FOUNDATION FOR THE ADVANCEMENT OF MILITARY MEDICINE, Rockville, MD 20852 (US)
(72) Inventor: SRIVASTAVA, Shiv, Potomac, MD 20814 (US); MOUL, Judd, W., Bethesda, MD 20817 (US); XU, Linda, L., Rockville, MD 20850 (US)
(74) Representative: Baldock, Sharon Claire
(86) International application number: PCT/US2003/013401
(87) International publication number: WO 2003/095611

(56) References cited:
- WO-A-00/50454
- WO-A-00/52022
- WO-A-02/30268
- US-B1- 6 268 377
- US-B1- 6 303 324
- US-B1- 6 566 130
- XU ET AL: 'A novel androgen-regulated gene, PMEPA1, located on chromosome 20q13 exhibits high level expression in prostate' GENOMICS vol. 66, 2000, pages 257 - 263, XP002257424

### SEQUENCE LISTING

<110> HENRY M. JACKSON FOUNDATION FOR THE ADVANCEMENT OF
   MILITARY MEDICINE
   SRIVASTAVA, SHIV
   XU, LINDA L.
   MOUL, JUDD W.
<120> ANDROGEN-REGULATED PMEPA1 GENE AND METHODS OF USING THE SAME TO INHIBIT CANCER CELL GROWTH
<130> 4995.0060-00304
<140>
   <141>
<150> 60/378,949
   <151> 2002-05-10
<160> 16
<170> PatentIn Ver. 2.1
<210> 1
   <211> 759
   <212> DNA
   <213> Homo sapiens
<400> 1
   atggcggagc tggagtttgt tcagatcatc atcatcgtgg tggtgatgat ggtgatggtg 60
   gtggtgatca cgtgcctgct gagccactac aagctgtctg cacggtcctt catcagccgg 120
   cacagccagg ggcggaggag agaagatgcc ctgtcctcag aaggatgcct gtggccctcg 180
   gagagcacag tgtcaggcaa cggaatccca gagccgcagg tctacgcccc gcctcggccc 240
   accgaccgcc tggccgtgcc gcccttcgcc cagcgggagc gcttccaccg cttccagccc 300
   acctatccgt acctgcagca cgagatcgac ctgccaccca ccatctcgct gtcagacggg 360
   gaggagcccc caccctacca gggcccctgc accctccagc ttcgggaccc cgagcagcag 420
   ctggaactga accgggagtc ggtgcgcgca cccccaaaca gaaccatctt cgacagtgac 480
   ctgatggata gtgccaggct gggcggcccc tgccccccca gcagtaactc gggcatcagc 540
   gccacgtgct acggcagcgg cgggcgcatg gaggggccgc cgcccaccta cagcgaggtc 600
   atcggccact acccggggtc ctccttccag caccagcaga gcagtgggcc gccctccttg 660
   ctggagggga cccggctcca ccacacacac atcgcgcccc tagagagcgc agccatctgg 720
   agcaaagaga aggataaaca gaaaggacac cctctctag                       759
<210> 2
   <211> 252
   <212> PRT
   <213> Homo sapiens
<400> 2
   Met Ala Glu Leu Glu Phe Val Gln Ile Ile Ile Ile Val Val Val Met
     1               5                  10                  15
   Met Val Met Val Val Val Ile Thr Cys Leu Leu Ser His Tyr Lys Leu
                20                  25                  30
   Ser Ala Arg Ser Phe Ile Ser Arg His Ser Gln Gly Arg Arg Arg Glu
            35                  40                  45
   Asp Ala Leu Ser Ser Glu Gly Cys Leu Trp Pro Ser Glu Ser Thr Val
   50 55 60
   Ser Gly Asn Gly Ile Pro Glu Pro Gln Val Tyr Ala Pro Pro Arg Pro
   65 70 75 80
   Thr Asp Arg Leu Ala Val Pro Pro Phe Ala Gln Arg Glu Arg Phe His
   85 90 95
   Arg Phe Gln Pro Thr Tyr Pro Tyr Leu Gln His Glu Ile Asp Leu Pro
   100 105 110
   Pro Thr Ile Ser Leu Ser Asp Gly Glu Glu Pro Pro Pro Tyr Gln Gly
   115 120 125
   Pro Cys Thr Leu Gln Leu Arg Asp Pro Glu Gln Gln Leu Glu Leu Asn
   130 135 140
   Arg Glu Ser Val Arg Ala Pro Pro Asn Arg Thr Ile Phe Asp Ser Asp
   145 150 155 160
   Leu Met Asp Ser Ala Arg Leu Gly Gly Pro Cys Pro Pro Ser Ser Asn
   165 170 175
   Ser Gly Ile Ser Ala Thr Cys Tyr Gly Ser Gly Gly Arg Met Glu Gly
   180 185 190
   Pro Pro Pro Thr Tyr Ser Glu Val Ile Gly His Tyr Pro Gly Ser Ser
   195 200 205
   Phe Gln His Gln Gln Ser Ser Gly Pro Pro Ser Leu Leu Glu Gly Thr
   210 215 220
   Arg Leu His His Thr His Ile Ala Pro Leu Glu Ser Ala Ala Ile Trp
   225 230 235 240
   Ser Lys Glu Lys Asp Lys Gln Lys Gly His Pro Leu
   245 250
<210> 3
   <211> 243
   <212> PRT
   <213> Mus sp.
<400> 3
   Ile Thr Glu Leu Glu Phe Val Gln Ile Val Val Ile Val Val Val Met
   1 5 10 15
   Met Val Met Val Val Met Ile Thr Cys Leu Leu Ser His Tyr Lys Leu
   20 25 30
   Ser Ala Arg Ser Phe Ile Ser Arg His Ser Gln Ala Arg Arg Arg Asp
   35 40 45
   Asp Gly Leu Ser Ser Glu Gly Cys Leu Trp Pro Ser Glu Ser Thr Val
   50 55 60
   Ser Gly Gly Met Pro Glu Pro Gln Val Tyr Ala Pro Pro Arg Pro Thr
   65                  70                  75                  80
   Asp Arg Leu Ala Val Pro Pro Phe Ile Gln Arg Ser Arg Phe Gln Pro
                   85                  90                  95
   Thr Tyr Pro Tyr Leu Gln His Glu Ile Ala Leu Pro Pro Thr Ile Ser
               100                 105                 110
   Leu Ser Asp Gly Glu Glu Pro Pro Pro Tyr Gln Gly Pro Cys Thr Leu
           115                 120                 125
   Gln Leu Arg Asp Pro Glu Gln Gln Leu Glu Leu Asn Arg Glu Ser Val
       130                 135                 140
   Arg Ala Pro Pro Asn Arg Thr Ile Phe Asp Ser Asp Leu Ile Asp Ser
   145                 150                 155                 160
   Thr Met Leu Gly Gly Pro Cys Pro Pro Ser Ser Asn Ser Gly Ile Ser
                   165                 170                 175
   Ala Thr Cys Tyr Ser Ser Gly Gly Arg Met Glu Gly Pro Pro Pro Thr
               180                 185                 190
   Tyr Ser Glu Val Ile Gly His Tyr Pro Gly Ser Ser Phe Gln His Gln
           195                 200                 205
   Gln Ser Asn Gly Pro Ser Ser Leu Leu Glu Gly Thr Arg Leu His His
       210                 215                 220
   Ser His Ile Ala Pro Leu Glu Asn Lys Glu Lys Glu Lys Gln Lys Gly
   225                 230                 235                 240
   His Pro Leu
<210> 4
   <211> 21
   <212> DNA
   <213> Artificial Sequence
<220>
   <223> Description of Artificial Sequence: Primer
<400> 4
   gctgctggag aactgaaggc g 21
<210> 5
   <211> 22
   <212> DNA
   <213> Artificial Sequence
<220>
   <223> Description of Artificial Sequence: Primer
<400> 5
   gtgtcctttc tgtttatcct tc 22
<210> 6
   <211> 27
   <212> DNA
   <213> Artificial Sequence
<220>
   <223> Description of Artificial Sequence: Primer
<400> 6
   aagcttgctg ctggagaact gaaggcg 27
<210> 7
   <211> 25
   <212> DNA
   <213> Artificial Sequence
<220>
   <223> Description of Artificial Sequence: Primer
<400> 7
   gaattcggtg tcctttctgt ttatc 25
<210> 8
   <211> 27
   <212> DNA
   <213> Artificial Sequence
<220>
   <223> Description of Artificial Sequence: Primer
<400> 8
   gcaggatccc aaccagatgc tgcttgc 27
<210> 9
   <211> 28
   <212> DNA
   <213> Artificial Sequence
<220>
   <223> Description of Artificial Sequence: Primer
<400> 9
   gcagaattct tttgtaatcc ctggagta 28
<210> 10
   <211> 27
   <212> DNA
   <213> Artificial Sequence
<220>
   <223> Description of Artificial Sequence: Primer
<400> 10
   gcaaagcttg tccggtttgc tggaagc 27
<210> 11
   <211> 31
   <212> DNA
   <213> Artificial Sequence
<220>
   <223> Description of Artificial Sequence: Primer
<400> 11
   gcagaattcc ctttttgttc ttattggtga c 31
<210> 12
   <211> 18
   <212> DNA
   <213> Artificial Sequence
<220>
   <223> Description of Artificial Sequence: Primer
<400> 12
   catgatcccc gagctgct 18
<210> 13
   <211> 23
   <212> DNA
   <213> Artificial Sequence
<220>
   <223> Description of Artificial Sequence: Primer
<400> 13
   tgatctgaac aaactccagc tcc 23
<210> 14
   <211> 23
   <212> DNA
   <213> Artificial Sequence
<220>
   <223> Description of Artificial Sequence: Primer
<400> 14
   aggcggacag tctcctgcga aac 23
<210> 15
   <211> 4
   <212> PRT
   <213> Artificial Sequence
<220>
   <223> Description of Artificial Sequence: Synthetic motif
<400> 15
   Pro Pro Pro Tyr
    1
<210> 16
   <211> 4
   <212> PRT
   <213> Artificial Sequence
<220>
   <223> Description of Artificial Sequence: Synthetic motif
<400> 16
   Pro Pro Thr Tyr
     1

## Description

### FIELD OF THE INVENTION

The present invention relates to tumor suppressor genes, and in particular, PMEPA1 genes, and the proteins encoded by these genes, including variants and/or analogs thereof. More particularly, the present invention is based in part on the discovery that PMEPA1 polypeptides inhibit cancer cell growth. Methods of inhibiting the growth of a cancer cell are provided. Methods of diagnosing or prognosing prostate cancer are also provided.

### BACKGROUND

Prostate cancer (CaP) is the most common malignancy in American men and the second leading cause of cancer deaths [Jernal et al., Cancer statistics, 2002. CA Cancer J Clin. 52:23-47,2002]. Despite ongoing research, much remains unknown about specific genetic alterations associated with CaP onset and progression [Augustus et al., The molecular phenotype of the malignant prostate. In S. Srivastava, D. E. Henson, and A. Gazden (eds), Molecular pathology of early cancer, pp 321-340. IOS press, Amsterdam, 1999; Moul et al. 1994. Molecular biology of CaP. Oncogenes and tumor suppressor genes. Current Clinical Oncology: CaP. (Eds. Dawson, N.A. and Vogelzang, N.J.), Wiley-Liss Publications, 19-46; Lalani et al., Cancer and Mets. Rev., 16:29-66, 1997; Shi et al., World J. Urol.; 14, 318-328, 1996; Heidenberg et al., Urology, 48:971-979, 1996; Bova et al., World J. Urol., 14:338-346, 1996; and Issacs et al., Prostate Cancer: The Genetic Basis of Human Cancer. Eds. Vogelstein B, and Kinzler KW, McGraw-Hill Companies, Inc., pp. 653-660, 1998]. Therefore, identification and characterization of genetic alterations associated with the onset and progression of prostate cancer is beneficial in understanding the biology and clinical course of the disease.

While alterations of tumor-suppressor genes and oncogenes are important in prostate tumorigenesis, hormonal mechanisms also play equally important roles in the development of prostate cancer. One such hormone, androgen, is involved in the process of prostate growth and development. Androgen binds to an androgen receptor on the cell surface of prostate cells. Androgen binding initiates a cascade of intracellular signaling that leads to the activation, or transcription, of specific target genes called androgen-regulated genes (ARGs) [Augustus et al., The molecular phenotype of the malignant prostate. In S. Srivastava, D. E. Henson, and A. Gazden (eds), Molecular pathology of early cancer, pp 321-340. IOS press, Amsterdam, 1999; Gelman E, J. Clin. Oncol., 20:3001-3015, 2002; Grossmann et al., J. Natl. Cancer Inst., 93:1687-1697, 2001]. These ARGs encode proteins with diverse functions that affect cell growth and differentiation. Androgen has been shown to induce proliferation and differentiation of prostate cells. In addition, depriving prostate cells of androgen has been shown to induce cell death. Thus, androgen regulation of specific target genes appears to play a role in both normal prostate growth and the development of prostate cancer.

In light of these studies, a systematic and comprehensive analysis of the ARGs and their biologic functions should provide a better understanding of how genetic alterations contribute to the onset and progression of prostate cancer and how to use these ARGs to better diagnose and treat prostate cancer.

WO 00/52022 discloses a polypeptide designated Tango 261 and the polynucleotide encoding this polypeptide which are expressed in a number of tissue types and which can be used for the treatment of prostate disorders.

WO 02/30268 describes genes whose expression is up or down regulated in prostate cancer. It further describes methods of diagnosing and treating prostate cancer and of identifying modulators for prostate cancer.

WO 00/50454 discloses nucleic acid sequences and peptides designated AS3 and anti-AS3 antibodies and methods of modulating cell proliferation and determining the presence of an increased risk of developing prostate cancer.

### SUMMARY OF THE INVENTION

Previously we described the identification of a novel androgen-regulated gene that exhibits abundant expression in prostate tissue (US2004/0092469). The novel gene has been designated PMEPA1.

Here we report the further evaluation of the biologic functions of PMEPA1. The present invention is based in part on the discovery that PMEPA1 inhibits cancer cell growth. Thus, in one embodiment, the invention provides a method of inhibiting the growth of a prostate cancer cell, comprising administering a polypeptide comprising SEQ ID NO:2 to the prostate cancer cell, in an amount effective to inhibit growth of the prostate cancer cell. The polypeptide may be administered directly to the cell or indirectly using a vector containing a polynucleotide sequence that encodes a polypeptide comprising SEQ ID NO:2. These methods include therapeutic methods of treating prostate cancer.

In another aspect, the invention provides variants of the PMEPA1 polypeptide having one or more mutation and/or deletion in one or both of the PY motifs of PMEPA1, as discussed in further detail below. Such mutations reduce the cell growth inhibitory effects of PMEPA1. These PMEPA1 variants can be used, for example, to define cellular proteins through which PMEPA1 interacts, directly or indirectly, to mediate cell growth inhibitory functions.

In a still further embodiment, the invention provides the polynucleotides that encode the PMEPA1 variants, as well as methods (as described above for a polypeptide comprising SEQ ID NO:2) of using these variants, for example, to diagnose, prognose or inhibit prostrate cancer cell growth.

In another embodiment, the invention provides a method for diagnosing or prognosing prostate cancer by measuring the levels of PMEPA1 expression. Thus, a method of the invention comprises detecting the expression levels of a polynucleotide in a biological sample from a patient, wherein the polynucleotide encodes a PMEPA1 polypeptide, and determining the probability of prostate cancer development or progression, wherein reduced expression of the polynucleotide in the biological sample correlates with an increased probability of cancer development or progression. For example, in a method of prognosing prostate cancer, reduced expression of PMEPA1 correlates with advanced stages of prostate cancer, such as non-organ confined tumors. The reduced expression may be measured by comparing PMEPA1 expression levels to a control sample. Alterntatively, a threshold value of PMEPA1 expression can be selected. In this case, if the PMEPA1 expression level is less than the threshold value, it is considered reduced. The threshold value can be determined using known techniques. For example, the value can be determined from the PMEPA1 RNA copy number or the cycle threshold value (cT). In one embodiment, the biological sample is obtained from a patient with prostate cancer who has undergone therapy, such as prostate surgery. In this way, PMEPA1 serves as a biological marker to predict cancer progression and/or to measure the therapeutic efficacy of treatment. In another embodiment, the polynucleotide encodes a polypeptide having the amino acid sequence of SEQ ID NO:2. In other embodiments, the polynucleotide encodes a variant of SEQ ID NO:2, that are at least 95% identical to SEQ ID NO:2 and inhibit prostate cancer cell growth.

In another embodiment, the method of diagnosing or prognosing prostate cancer comprises detecting the expression levels in a biological sample of a PMEPA1 polypeptide, and determining the probability of prostate cancer development or progression, wherein reduced expression of the polypeptide in the biological sample correlates with an increased probability of cancer development or progression. As discussed above, the reduced expression may be measured by comparing PMEPA1 expression to a control sample and/or to a threshold value, which may be predetermined. In one embodiment, the biological sample is obtained from a patient with prostate cancer who has undergone therapy, such as prostate surgery. In another embodiment, the polypeptide has the amino acid sequence of SEQ ID NO:2. In other embodiments, the polypeptides are at least 95% identical to SEQ ID NO:2 and inhibit prostate cancer cell growth. PMEPA1 polypeptides can be detected, for example, using antibodies that specifically bind to PMEPA1 polypetide.

The invention can be used in a method of screening to detect compounds that specifically bind to a polypeptide comprising SEQ ID NO:2. The method comprises contacting the polypeptide with a test compound, measuring the binding between the polypeptide and the compound, and identifying the compound that specifically binds to the polypeptide. The method may involve identifying a compound that increases the expression of PMEPA1 in a cell, particularly a cancer cell, such as a prostate cancer cell.

Additional aspects of the invention will be set forth in part in the description following, and in part will be understood from the description, or may be learned by practice of the invention.

### BRIEF DESCRIPTION OF THE DRAWINGS

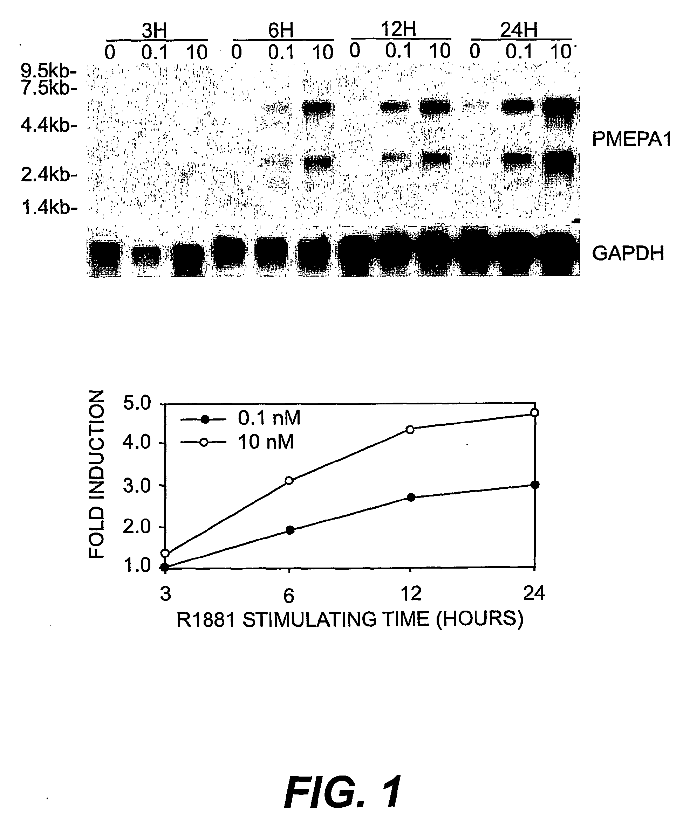

Figure 1 shows the androgen-dependent expression of PMEPA1. It represents a northern blot analysis using PMEPA1 probe with mRNA derived from LNCaP cells with or without R1881 (androgen) treatment for various durations. In comparison to androgen dependent LNCaP cells, androgen-independent prostate cancer cell lines DU145 and PC3 expressed very low to undetectable levels of PMEPA1 (not shown).

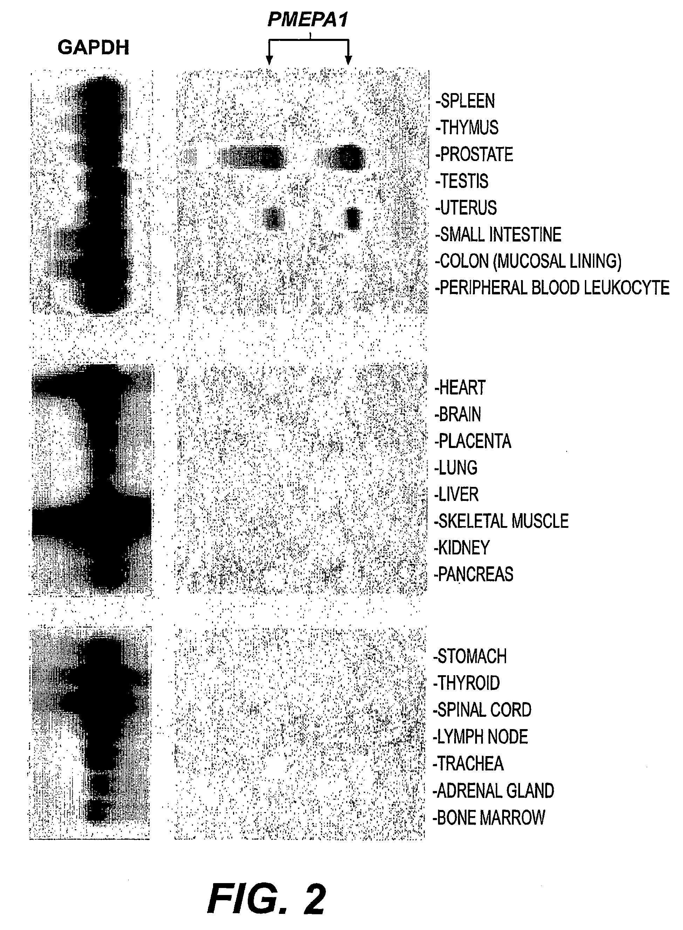

Figure 2 represents multiple tissue northern blots that were hybridized with PMEPA1 and GAPDH probes and shows that PMEPA1 is expressed at high levels in the prostate. The arrows indicate the two variants of PMEPA1 transcripts. Of the 23 tissues tested, prostate showed the highest expression of PMEPA1.

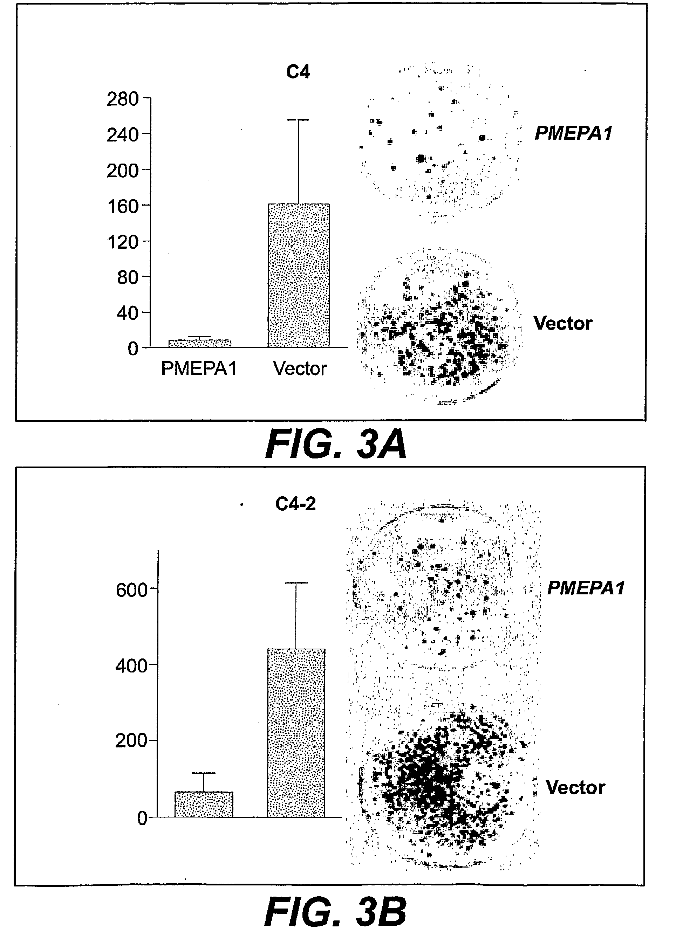

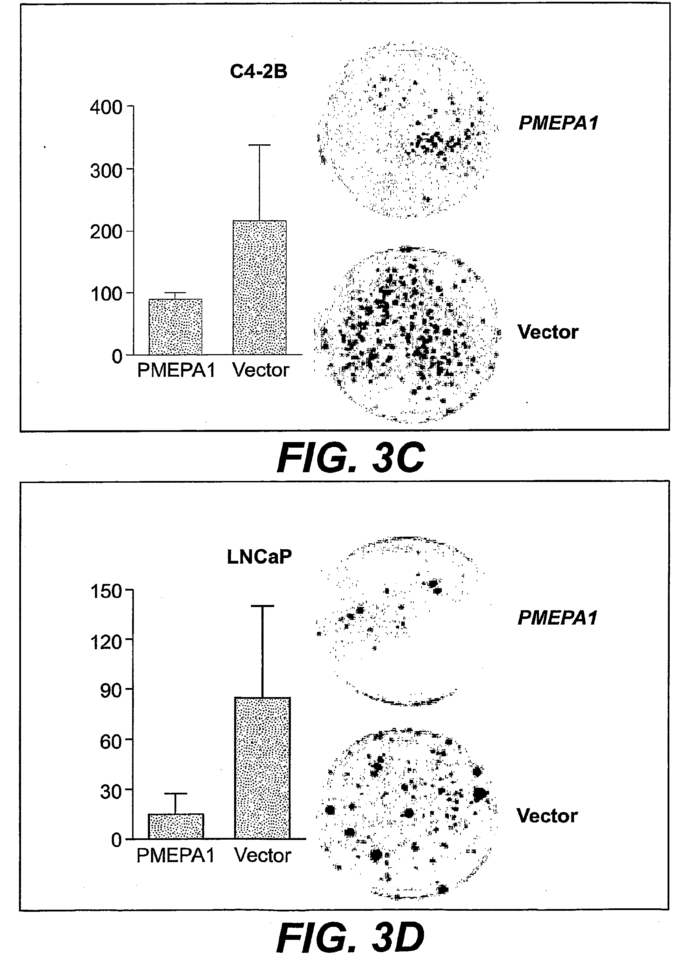

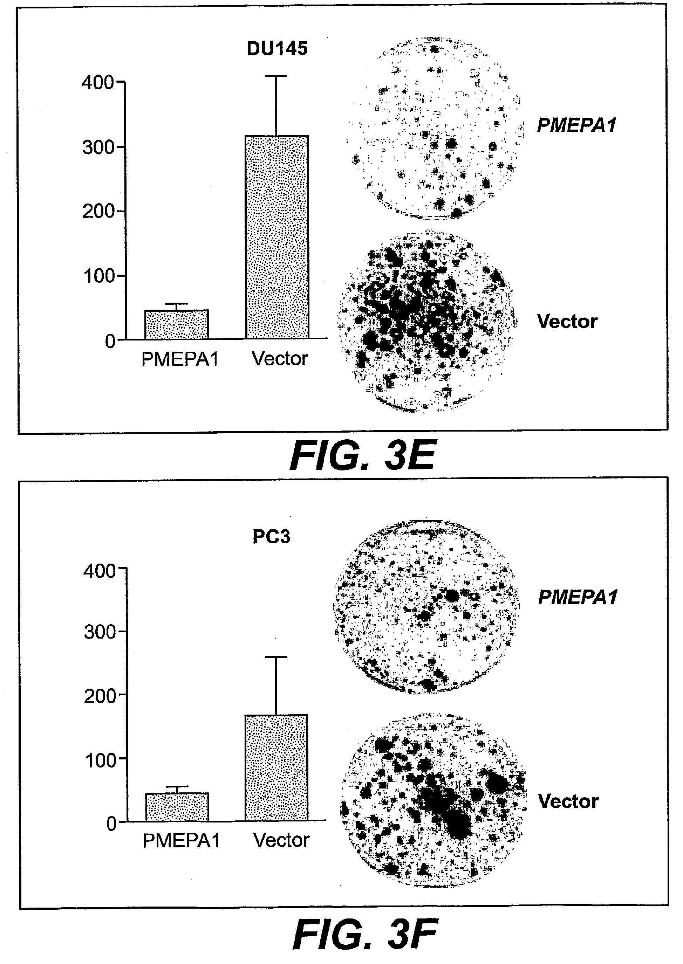

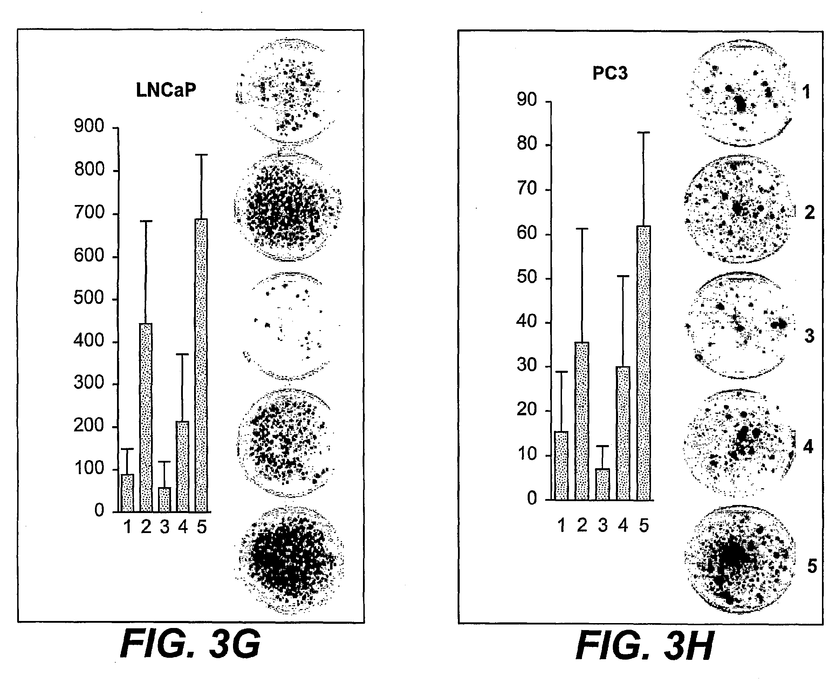

Figures 3A-H show the effect of PMEPA1 on colony formation. Prostate tumor cell lines: C4 (Fig. 3*A*), C4-2 (Fig. 3*B*), C4-2B (Fig. 3*C*), LNCaP (Fig. 3*D*), DU145 (Fig. 3*E*), and PC3 (Fig. 3*F*) were transfected with 3 µg of each of PMEPA1-V5-pcDNA3.1 (PMEPA1) and pcDNA3.1 vector (Vector) in triplicate sets. In a separate experiments LNCaP (Fig. 3*G*) and PC3 (Fig. 3*H*) cells were transfected with control vector or expression vectors encoding wt-PMEPA1 or PMEPA1-PY mutants: 1. PMEPA1-V5-pcDNA3.1, 2. PMEPA1*-PY1m*-pcDNA3.1, 3. PMEPA1-*PY2m*-pcDNA3.1, 4. PMEPA1-*PY1m*/*PY2m*-pcDNA3.1, and 5. pcDNA3.1). Transfected cells were selected for plasmid-containing cells with G418 for 3 weeks and surviving cells were fixed and stained with crystal violet. In each experiment, the number of colonies per dish were counted and displayed as histograms, representing the mean number of colonies ± SD of the triplicate sets. For each cell line, a photograph of one dish of cells treated with 3 µg of each plasmid is also shown.

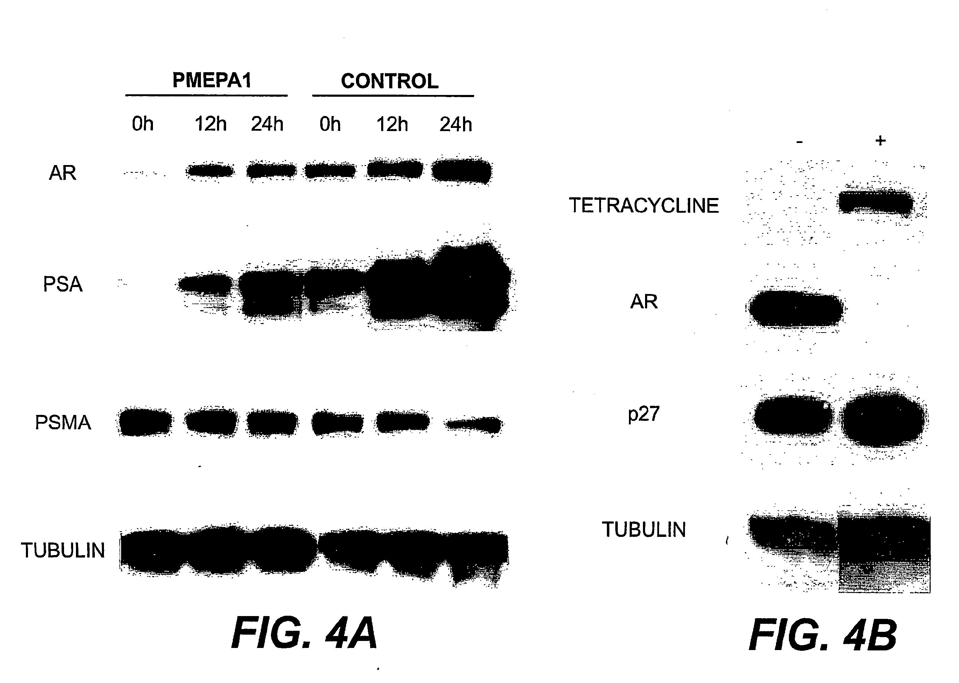

Figure 4A shows PMEPA1-mediated down regulation of androgen receptor and its functional consequences on androgen receptor regulated genes. LNCaP cells stably transfected with PMEPA1-GFP and pEGFP (control) plasmids were cultured in medium with cFBS for 5 days and then were stimulated with R1881 at 0.1 nM. Cells were harvested for Western blotting at 0 h, 12 h and 24 h after androgen stimulation. Antibodies against androgen receptor, PSA, PSMA and tubulin were used to detect corresponding proteins on Western Blots.

Figure 4B shows that PMEPA1 does not reduce androgen receptor expression through a non-specific, PMEPA1-induced effect on the ubiquitin-proteasome pathway. Stable PMEPA1-GFP-Tet -LNCaP transfectants (Tet-off system) were cultured in proper medium with or without tetracycline for 10 days and were applied for immunoblotting. Antibodies against androgen receptor, GFP, p27 and tubulin were used to detect the corresponding proteins.

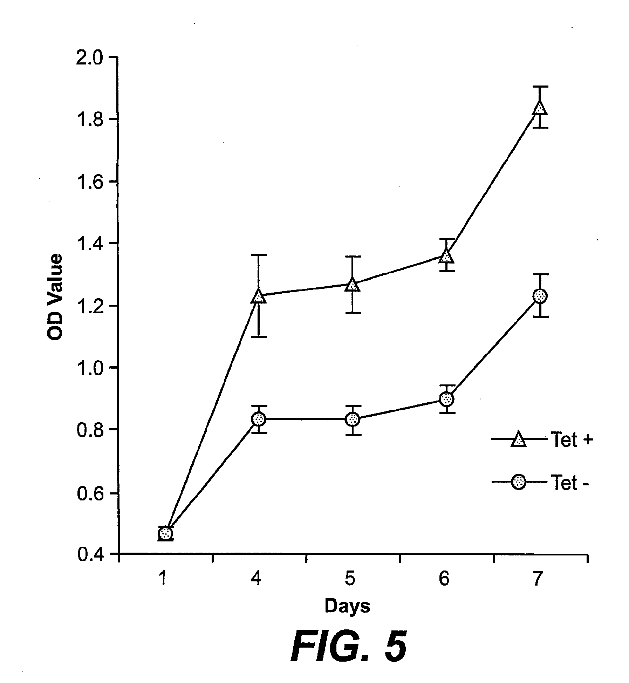

Figure 5 shows the effect of PMEPA1 on cell proliferation. Stable PMEPA1-GFP-Tet -LNCaP transfectants were seeded in 96-well plates with or without 1 µg/ml of tetracycline in the medium. The cell proliferation was measured using the CellTiter 96 Aqueous One Solution kit at the indicated time. Tet + and Tet - denote the cell culture medium with or without tetracycline, respectively. The OD values reflecting the cell numbers are significantly different (p<0.01) between the two groups except on day one.

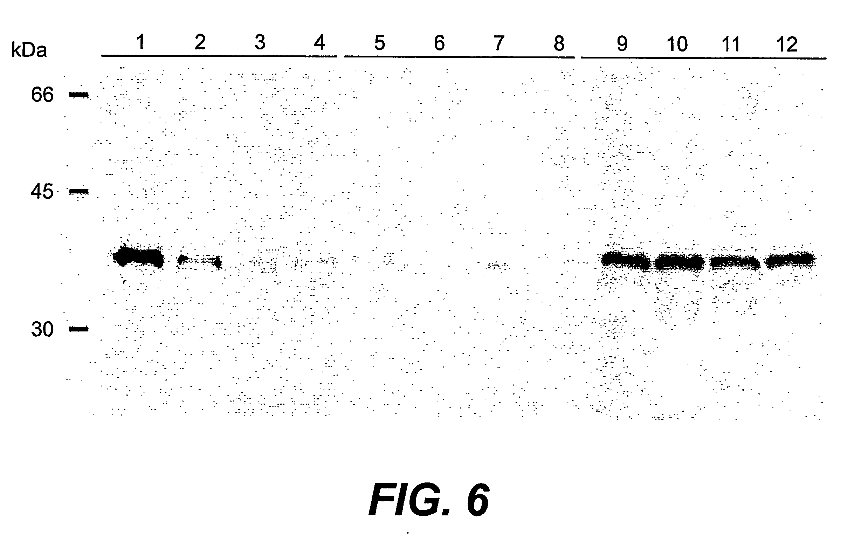

Figure 6 defines binding of PMEPA1 to NEDD4 proteins. The *in vitro* transcription/translation products ([³⁵S]Methionine-labeled lysates) derived from expression plasmids: PMEPA1-V5-pcDNA3.1 (Lanes 1, 5), PMEPA1-*PY1m*-pcDNA3.1 (Lanes 2, 6), PMEPA1-*PY2m*-pcDNA3.1 (Lanes 3, 7), and PMEPA1-*PY1m*/*PY2m*-pcDNA3.1 (Lanes 4, 8) were incubated with GST-NEDD4-WW- Sepharose beads (Lanes 1-4) or control GST beads (Lanes 5-8) and [³⁵S] Methionine labeled proteins bound to GST-NEDD4-WW-Sepharose beads were solublized in sample buffer and were resolved by SDS-PAGE gel. Equal amounts of [³⁵S]Methionine lysates corresponding to samples in lanes 1-4 were run on SDS-PAGE gel without GST pull-down (Lane 9-12).

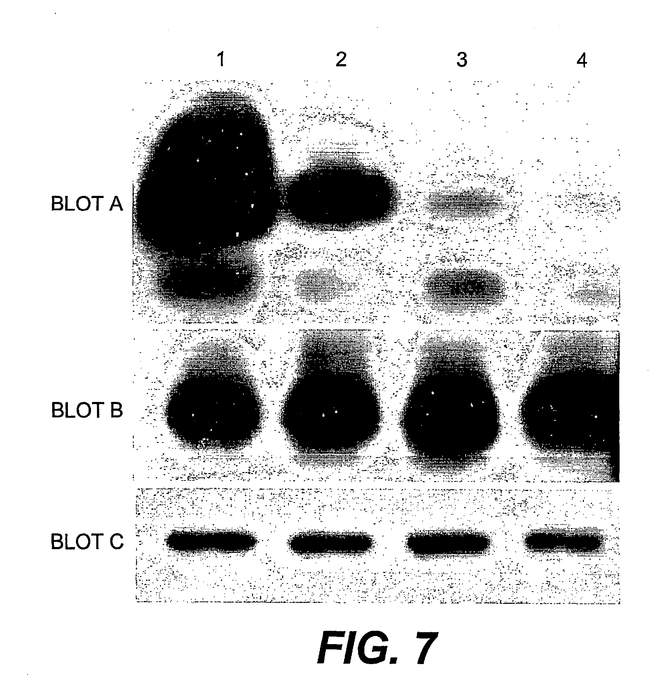

Figure 7 represents an immunoprecipitation assay. 293 cells were co-transfected with expression vectors encoding NEDD4-GFP and one of following fusion proteins: PMEPA1-V5 (Lane 1), PMEPA1-PY1m-V5 (Lane 2), PMEPA1-PY2m-V5 (Lane 3) or PMEPA1-PY1m/PY2m-V5 (Lane 4). The cell lysates from each group were immunoprecipitated with anti-GFP antibody then subjected to immunoblotting (*blot a*). Cell lysates from each group without immunoprecipitation were also processed for immunoblotting (*blots b and c*) to serve as a control. *Blots a and b* were detected by anti-V5 antibody and *blot c* was detected by anti-GFP antibody.

### DETAILED DESCRIPTION OF THE INVENTION

PMEPA1 was discovered in our laboratory through Serial Analysis of Gene Expression (SAGE) as the gene in prostate cancer cells that is most induced by androgen [Xu et al., Int. J. Cancer, 92: 322-328, 2001; Xu et al., Genomics, 66:257-263, 2000]. The expression of PMEPA1 is regulated by androgen in a dose- and time-dependent manner and is highly expressed in the prostate in comparison to other organs US 2004 10092469.

As summarized below and explained in further detail in the Examples that follow, our evaluation of PMEPA1 indicates it is a prostate-abundant androgen regulated gene with roles in cell growth control and tumorigenesis. Loss or reduced PMEPA1 expression in prostate cancer correlates with a higher risk or probability of prostate tumorigenesis or progression (e.g., advanced stages of prostate cancer, such as non-organ defined cancer, where tumors extend beyond the prostate gland), particularly after surgery as primary therapy. Thus, alterations in the level, expression, and activity of PMEPA1 and/or its encoded polypeptide provides useful information about the clinical behavior of prostate cancer. Part of our evaluation involved a PMEPA1 protein sequence homology search that showed 83% identity to a recently reported gene, N4WBP4 (Example 3). N4WBP4 encodes a NEDD4 WW domain binding protein with two PY motifs that is expressed in mouse embryo [Jolliffe et al., Biochem. J., 351: 557-565, 2000]. The PY motif is a proline-rich peptide sequence with a consensus PPXY sequence (where X can be any amino acid) that can bind to proteins with WW domains [Jolliffe et al., Biochem. J., 351: 557-565, 2000; Harvey Ket al., Trends Cell Biol., 9: 166-169, 1999; Hicke L, Cell, 106: 527-530, 2001; Kumar et al., Biochem. Biophys. Res. Commun., 185: 1155-1161, 1992; Kumar et al., Genomics, 40: 435-443, 1997; Sudol M, Trends Biochem. Sci., 21: 161-163, 1996; Harvey et al., J. Biol. Chem., 277: 9307-9317, 2002; and Brunschwig et al., Cancer Res., 63: 1568-1575, 2003]. NEDD4 was originally identified as a developmentally regulated gene in mice and is a ubiquitin-protein ligase (E3) that is involved in the ubiquitin-dependent proteasome-mediated protein degradation pathway. Further studies revealed that NEDD4 is implicated in diverse cellular functions, such as regulation of membrane channels and permeases, endocytosis, virus budding, cell cycle, transcription and protein trafficking [Harvey et al., Trends Cell Biol., 9: 166-169, 1999; Hicke L, Cell, 106: 527-530, 2001]. The WW domain present in the NEDD4 protein is a module with two highly conserved tryptophans that bind to several target proteins containing a PY motif.

As explained in Example 4, we discovered that PMEPA1 is a NEDD4 binding protein and that the binding of PMEPA1 to NEDD4 is mediated by the PY motifs of PMEPA1. Mutating the PY motifs significantly reduces the binding of PMEPA1 to NEDD4. In addition, the homology of PMEPA1 to the NEDD4-binding protein indicates that PMEPA1 may also regulate protein turnover via ubiquitinylation and proteasome pathways in the cell. This is further supported by our observation that PMEPA1 localizes to the Golgi apparatus (Example 6).

Further, we recently found that PMEPA1 expression in LNCaP cells down regulates androgen receptor protein and modulates the expression of genes that are transcriptionally regulated by androgen receptor (Example 5). This shows that PMEPA1 functions in androgen receptor regulation.

Our data also show that PMEPA1 inhibits the growth of prostate cancer cells (Example 7). More specifically, the coding region of PMEPA1 was inserted into an expression vector and transfected into 293 cell (kidney) and LNCaP cells (prostate cancer). Cell proliferation and cell cycle analysis showed that there was no difference between PMEPA1 overexpressed 293 cell and control vector transfected 293 cells. However LNCaP cells overexpressing PMEPA1 exhibited significant cell growth inhibition. Similar growth inhibition was observed in other prostate cancer cell lines.

In addition, in a quantitative evaluation of PMEPA1 expression in primary prostate cancers, we found that 40 of 62 (64.5%) matched prostate specimens exhibited decreased expression of PMEPA1 in tumor tissues, indicating a correlation between reduced PMEPA1 expression and prostate tumorigenesis (Example 8). When these expression patterns were stratified by organ confined and non-organ confined tumors, a higher percentage of patients exhibited reduced expression of PMEPA1 in non-organ confined tumor (68%) vs. organ-confined tumor (44%), indicating that reduced PMEPA1 expression correlates with an increased probability of advanced prostate cancer.

### Definitions

The term "PMEPA1 gene" refers to a polynucleotide sequence encoding a PMEPA1 polypeptide having functional and structural characteristics as set out in the definition of PMEPA1 polypeptide.

The term "PMEPA1 polypeptide," "PMEPA protein", or "PMEPA1" for short where the context permits, refers to a polypeptide substantially identical to SEQ ID NO:2. The definition further encompasses PMEPA1 variants, analogs, e.g., orthologues and homologues, as well as functionally equivalent fragments of PMEPA1, variants, and analogs, the expression of which in cancerous cells inhibits cell growth. Example 7, discussed below, provides, by way of example, an assay for determining whether PMEPA1 expression inhibits tumor growth. Other standard methods known in the art may also be used.

The term "polypeptide" is used interchangeably with the terms "peptide" and "protein" and refers to any chain of amino acids, regardless of length or posttranslational modification (e.g., glycosylation or phosphorylation), or source (e.g., species).

The phrase "substantially identical," or "substantially as set out," means that a relevant sequence is at least 70%, 75%, 80%, 85%, 90%, 95%, 97%, 98%, or 99% identical to a given sequence. By way of example, such sequences may be allelic variants, sequences derived from various species, or they may be derived from the given sequence by truncation, deletion, amino acid substitution and/or addition. For polypeptides, the length of comparison sequences will generally be at least 20, 30, 50, 100 or more amino acids. For nucleic acids, the length of comparison sequences will generally be at least 50, 100, 150, 300, or more nucleotides. Percent identity between two sequences is determined by standard alignment algorithms such as, for example, Basic Local Alignment Tool (BLAST) described in Altschul et al. (1990) J. Mol. Biol., 215:403-410, the algorithm of Needleman et al. (1970) J. Mol. Biol., 48:444-453, or the algorithm of Meyers et al. (1988) Comput. Appl. Biosci., 4:11-17.

The term "androgen receptor" refers to a protein that binds to the male hormone testosterone.

The term "diagnose" means to detect, identify, monitor, or distinguish a disease or condition.

The term "prognose" means to define the risk of disease progression.

The term "correlates" means to establish or demonstrate as having a causal, complementary, parallel, or reciprocal relationship or qualitative and/or quantitative correspondence between two entities, e.g., between PMEPA1 expression and disease development or progression.

The phrase "inhibits the growth of a cancer cell" refers to a decrease in cell growth in the presence of a PMEPA1 polypeptide, relative to the cell growth in the absence of the PMEPA1 polypeptide. Alternatively, if a cell has a basal level of PMEPA1 polypeptide expression, the phrase "inhibits the growth of a cancer cell" refers to a decrease in cell growth in the presence of increased levels of PMEPA1 polypeptide, relative to cell growth in the presence of the basal level of PMEPA1 polypeptide. Cell growth can be measured using conventional assays, such as the colony-forming assay described in the examples.

The phrase "reduce the expression of an androgen receptor" refers to a decrease in expression of androgen receptor protein or nucleic acid in the presence of a PMEPA1 polypeptide, relative to the the expression in the absence of the PMEPA1 polypeptide. Alternatively, if a cell has a basal level of PMEPA1 polypeptide expression, the phrase "reduce the expression of an androgen receptor" refers to a decrease in expression of androgen receptor protein or nucleic acid in the presence of increased levels of PMEPA1 polypeptide, relative to the expression of androgen receptor in the presence of the basal level of PMEPA1 polypeptide.

The terms "specific interaction," "specific binding," or the like, mean that two molecules form a complex that is relatively stable under physiologic conditions. The term is also applicable where, e.g., an antigen-binding domain is specific for a particular epitope, which is carried by a number of antigens, in which case the specific binding member carrying the antigen-binding domain will be able to bind to the various antigens carrying the epitope. Specific binding is characterized by a high affinity and a low to moderate capacity. Nonspecific binding usually has a low affinity with a moderate to high capacity. Typically, the binding is considered specific when the affinity constant Kₐ is higher than 10⁶ M⁻¹, but the Kₐ may be higher than 10⁷ M⁻¹, and higher than 10⁸ M⁻¹ in alternative embodiments. If necessary, non-specific binding can be reduced without substantially affecting specific binding by varying the binding conditions. Such conditions are known in the art, and a skilled artisan using routine techniques can select appropriate conditions. The conditions are usually defined in terms of concentration of molecules, ionic strength of the solution, temperature, time allowed for binding, concentration of non-related molecules (e.g., serum albumin, milk casein), etc.

The term "detectably labeled" refers to any means for marking and identifying the presence of a molecule, e.g., an oligonucleotide probe or primer, a gene or fragment thereof, or a cDNA molecule. Methods for labeling a molecule are well known in the art and include, without limitation, radioactive labeling (e.g., with an isotope such as ³²P, ³⁵S, or ¹²⁵I) and nonradioactive labeling (e.g., chemiluminescent labeling).

The term "modulate" refers to the capability of a compound acting as either an agonist or an antagonist of a certain reaction or activity. The term modulate, therefore, encompasses the terms "activate" and "inhibit."

The term "modulatory compound" means any compound capable of "modulating" either PMEPA1 expression at the transcriptional, translational, or post-translational levels or modulating the biological activity of a PMEPA1 polypeptide. The term "activate," for example, refers to an increase in the expression of a gene or activity of a polypeptide, such as PMEPA1, in the presence of a modulatory compound, relative to the activity of the gene or the polypeptide in the absence of the same compound. The increase in the expression level or the activity is preferably at least about 10%, 20%, 30%, 40%, 50%, 60%, 70%, 80%, 90%, or higher. Analogously, the term "inhibit" refers to a decrease in the expression of a gene or activity of a polypeptide, such as PMEPA1, in the presence of a modulatory compound, relative to the activity of the gene or the polypeptide in the absence of the same compound. The decrease in the expression level or the activity is preferably at least about 10%, 20%, 30%, 40%, 50%, 60%, 70%, 80%, 90%, or higher. The expression level of the gene or the activity of the polypeptide can be measured as described herein or by techniques generally known in the art.

The term "treatment" is used interchangeably herein with the term "therapeutic method" and refers to both therapeutic treatment and prophylactic/preventative measures. The terms "treatment" or "treating" includes the application or administration of a therapeutic agent to a subject or to an isolated tissue or cell from a subject, who is afflicted with a disease, a symptom of disease, or a predisposition toward a disease, with the goal of curing, healing alleviating, relieving, altering, remedying, ameliorating, imrproving, or affecting the disease, a symptom of the disease, or the redisposition toward the disease. Those in need of treatment may include individuals already having a particular medical disorder as well as those who may ultimately acquire the disorder. Administration of an agent prophylactically can occur prior to the manifestations of the symptoms of an undesired disease or disorder, such that the disease or disorder is prevented or, alternatively, delayed in its progression. The prophylactic or preventive methods of the present invention can be carried out in a similar manner to the other therapeutic methiods described herein, although dosage and treatment regimens may differ.

The term "isolated" refers to a molecule that is substantially free of its natural environment. Any amount of that molecule elevated over the naturally occurring levels due to any manipulation, e.g., overexpression, partial purification, etc., is encompassed with the definition. With regard to partially purified compositions only, the term refers to an isolated compound is at least 50-70%, 70-90%, 90-95% (w/w), or more pure.

The term "effective dose," or "effective amount," refers to that amount of the compound that results in amelioration of symptoms in a patient or the production of a desired biological outcome, e.g., inhibition of cell proliferation. The effective amount can be determined as described in the subsequent sections.

The terms "polynucleotide," "oligonucleotide," "nucleic acid," and "DNA" are used interchangeably herein and refer to deoxyribonucleic acid (DNA), and, where appropriate, ribonucleic acid (RNA), or chimeric mixtures or variants or modified versions thereof. The term should also be understood to include nucleotide analogs, and single or double stranded polynucleotides. Examples of polynucleotides include, but are not limited to, plasmid DNA or fragments thereof, viral DNA or RNA, anti-sense RNA, etc. The term "plasmid DNA" refers to double stranded DNA that is circular.

The term "transgene" refers to a polynucleotide that, when introduced into a cell, is capable of being transcribed under appropriate conditions so as to confer a beneficial property to the cell, for example, expression of a therapeutically useful protein. For gene therapy, the transgene is selected based upon a desired therapeutic outcome. Where appropriate, the term "transgene" should be understood to include a combination of a coding sequence and optional noncoding regulatory sequences, such as a polyadenylation signal, a promoter, enhancers, repressors, etc.

The term "transfection" is used interchangeably with the terms "gene transfer," "transformation," and "transduction," and means the intracellular introduction of a polynucleotide. "Transfection efficiency" refers to the relative amount of the transgene taken up by the cells subjected to transfection. In practice, transfection efficiency is estimated by the amount of the reporter gene product expressed following the transfection procedure.

The term "vector" is used interchangeably with "transgene delivery vector," "expression vector," "expression module," "expression cassette," "expression construct," and where appropriate, "nucleic acid" and refers to viral or non-viral, prokaryotic or eukaryotic, DNA or RNA sequences that are capable of being transfected into a cell, referred to as "host cell," so that all or a part of the sequences is transcribed. It is not necessary for the transcript to be expressed. It is also not necessary for a vector to comprise a transgene having a coding sequence. Vectors are frequently assembled as composites of elements derived from different viral, bacterial, or mammalian genes. Vectors contain various coding and non-coding sequences such sequences coding for selectable markers, sequences that facilitate their propagation in bacteria, or one or more transcription units that are expressed only in certain cell types. For example, mammalian expression vectors often contain both prokaryotic sequences that facilitate the propagation of the vector in bacteria and one or more eukaryotic transcription units that are expressed only in eukaryotic cells. It will be appreciated by those skilled in the art that the design of the expression vector can depend on such factors as the choice of the host cell to be transformed, the level of expression of protein desired, etc.

A minimal sequence of a vector that is sufficient to initiate transcription of a coding sequence of a transgene inserted in the vector is referred to as "promoter." Promoters may be constitutive or inducible, and may be coupled with other regulatory sequences/elements which, when bound to appropriate intracellular regulatory factors, enhance ("enhancers") or repress ("repressors") promoter-dependent transcription. A promoter, enhancer, or repressor, is said to be "operably linked" to a transgene when such element(s) control(s) or affect(s) transgene transcription rate or efficiency. For example, a promoter sequence that is located proximally to the 5' end of a transgene coding sequence is usually operably linked with the transgene. As used herein, term "regulatory elements" is used interchangeably with "regulatory sequences" and refers to promoters, enhancers, and other expression control elements, or any combination of such elements.

The term "transfection agent" refers to substances that may facilitate the transfer of the polynucleotide across the cell wall and/or cell membrane. Typically, such compounds reduce the electrostatic charge of the cell surface and the polynucleotide itself, or increase the permeability of the cell wall and/or cell membrane.

As used herein the term "hybridization under defined conditions" describes conditions for hybridization and washes under which nucleotide sequences that are significantly identical or homologous to each other remain bound to each other. The conditions are such that sequences, which are at least 50, 100, 150, 300, or more nucleotides long and at least about 70%, 80%, 85%, 90%, or 95% identical remain bound to each other. The percent identity can be determined as described in Altschul et al. (1997) Nucleic Acids Res., 25: 3389-3402. Nonlimiting examples of low stringency and high stringency hybridization conditions are provided later in this application.

### Gene Isolation

Genomic DNA may be isolated by conventional techniques, e.g., by using the DNA of SEQ ID NO: 1 or a suitable fragment thereof as a probe, or as described in the Examples.

The invention provides nucleic acids that hybridize to the PMEPA1 cDNA of SEQ ID NO:1, encoding the polypeptide provided in SEQ ID NO:2.

In one embodiment, polymerase chain reaction (PCR) is used to amplify the desired sequence prior to selection. Oligonucleotide primers representing known PMEPA1 sequences can be used as primers. For example, fragments of SEQ ID NO:1 comprising at least about 17, 30, or 60 contiguous nucleotides generally can be used as primers. The sequence being amplified can include mRNA, or cDNA, or genomic DNA from any eukaryotic species. One can choose to synthesize several different degenerate primers and to vary the stringency of hybridization conditions in priming the PCR redactions in order to allow for greater or lesser degrees of nucleotide sequence similarity between the known PMEPA1 nucleotide sequence and the nucleic acid homologue being isolated. For cross-species hybridization, low stringency conditions are recommended, while for same species hybridization, moderately stringent conditions are recommended. After successful amplification of a segment of a PMEPA1 sequence homologue, that segment may be molecularly cloned and sequenced, and utilized as a probe to isolate a complete cDNA or genomic clone. This permits the determination of the gene's complete nucleotide sequence. In this fashion, additional genes encoding PMEPA1 proteins may be identified without undue experimentation. Details of the procedures are described in Sambrook et al. Molecular Cloning, A Laboratory Manual, 3rd ed., Cold Spring Harbor Laboratory Press, Cold Spring Harbor, NY (www.molecularcloning.com); and DNA Cloning: A Practical Approach, Glover, ed., MRL Press, Ltd., Oxford, UK, 1985.

Once the polynucleotides containing all or a part of the PMEPA1 sequence are generated, identification of the specific DNA fragment containing the desired gene may be accomplished in a number of ways. For example, if an amount of a portion of a PMEPA1 (of any species) gene or its specific RNA, or a fragment thereof, is available and can be purified and labeled, the generated DNA fragments may be screened by nucleic acid hybridization to the labeled probe (Benton et al. (1977) Science, 196:180; Grunstein et al. (1975) Proc. Natl. Acad. Sci. U.S.A., 72:3961). Those DNA fragments with substantial homology to the probe will hybridize. It is also possible to identify the appropriate fragment by restriction enzyme digestion(s) and comparison of fragment sizes with those expected according to a known restriction map. Further selection can be carried out on the basis of the properties of the gene. Alternatively, the presence of the gene may be detected by assays based on the physical, chemical, or immunological properties of its expressed product. For example, cDNA clones can be selected based on properties of the protein they produce, e.g., similar or identical to PMEPA1 electrophoretic migration, isoelectric focusing behavior, proteolytic digestion maps, inhibition of cell proliferation, inhibition of androgen receptor expression, etc. If an antibody to PMEPA1 is available, the PMEPA1 protein may be identified by binding of detectably labeled antibody to the putative PMEPA1 in an ELISA (enzyme-linked immunosorbent assay)-type procedure.

PMEPA1 genes can also be identified and isolated by mRNA selection using nucleic acid hybridization followed by *in vitro* translation. In this procedure, fragments are used to isolate complementary mRNAs by hybridization. Such DNA fragments may represent available, purified PMEPA1 DNA of another species (e.g., Drosophila, mouse, human, etc.). Immunoprecipitation analysis or functional assays (e.g., inhibiting cancer cell growth, inhibiting androgen receptor expression, etc.) of the *in vitro* translation products of the isolated products identifies the mRNA and, therefore, the complementary DNA fragments that contain the desired sequences. In addition, specific mRNAs may be selected by adsorption of polysomes isolated from cells to immobilized antibodies specifically directed against PMEPA1 protein. A detectably labeled PMEPA1 cDNA can be synthesized using the selected mRNA (from the adsorbed polysomes) as a template. The mRNA or cDNA may then be used as a probe to identify the PMEPA1 DNA fragments from among other genomic DNA fragments.

Alternatives to isolating the PMEPA1 genomic DNA include, but are not limited to, chemically synthesizing the gene sequence itself from a known sequence or making cDNA to the mRNA which encodes the PMEPA1 protein. For example, RNA for cDNA cloning of the PMEPA1 gene can be isolated from cells which express PMEPA1. Other methods are possible and within the scope of the invention.

The identified and isolated gene can then be inserted into an appropriate cloning vector for subsequent cloning and expression as described in subsequent sections.

The cloned DNA or cDNA corresponding to the PMEPA1 gene can be analyzed by methods including but not limited to Southern hybridization (Southern (1975) J. Mol. Biol., 98:503-517), Northern hybridization (see, e.g., Freeman et al. (1983) Proc. Natl. Acad. Sci. U.S.A., 80:4094-4098), restriction endonuclease mapping (Maniatis (1982) Molecular Cloning: A Laboratory Manual, 3rd ed. Cold Spring Harbor, NY (www.molecularcloning.com), and DNA sequence analysis. Polymerase chain reaction (U.S. Patent Nos. 4,683,202; 4,683,195; and 4,889,818; Gyllenstein et al. (1988) Proc. Natl. Acad. Sci. U.S.A., 85:7652-7656; Ochman et al. (1988) Genetics, 120:621-623; Loh et al. (1989) Science, 243:217-220) followed by Southern hybridization with a PMEPA1-specific probe can allow the detection of the PMEPA1 gene in DNA from various cell types. Methods of amplification other than PCR are commonly known and can also be employed. In one embodiment, Southern hybridization can be used to determine the genetic linkage of PMEPA1. Northern hybridization analysis can be used to determine the expression of the PMEPA1 gene. Various cell types at various states of development or activity can be tested for PMEPA1 expression. The stringency of the hybridization conditions for both Southern and Northern hybridization can be manipulated to ensure detection of nucleic acids with the desired degree of relatedness to the specific PMEPA1 probe used. Modifications of these methods and other methods commonly known in the art can be used.

Restriction endonuclease mapping can be used to roughly determine the genetic structure of the PMEPA1 gene. Restriction maps derived by restriction endonuclease cleavage can then be confirmed by DNA sequence analysis.

DNA sequence analysis can be performed by any techniques known in the art, including but not limited to the method of Maxam and Gilbert (1980, Meth. Enzymol. 65:499-560), the Sanger dideoxy method (Sanger et al. (1977) Proc. Natl. Acad. Sci. U.S.A., 74:5463), the use of T7 DNA polymerase (U.S. Patent No. 4,795,699), or use of an automated DNA sequenator (e.g., Applied Biosystems, Foster City, CA).

### Nucleic Acids

Analysis of a 1,141 base pair PMEPA1 cDNA sequence revealed an open reading frame of 759 nucleotides having the following sequence:

Due to the degeneracy of the genetic code (i.e., more than one codon can encode the same amino acid) a PMEPA1 DNA sequence can vary from that shown in SEQ ID NO. 1 and still encode a polypeptide having the amino acid sequence of SEQ ID NO:2. Such variant DNA sequences can be allelic variants or "silent" mutations (e.g., occurring during PCR amplification), or can be deliberately introduced by mutagenesis of the native sequence.

PMEPA1 nucleic acids include isolated nucleic acid sequences selected from: (a) nucleic acid comprising the nucleotide sequence of SEQ ID NO:1; (b) a nucleic acid encoding the polypeptide of SEQ ID NO:2; (c) a nucleic acid capable of hybridization to the nucleic acid of (a) or (b) under defined conditions, wherein expression of the nucleic acid in LNCaP cells decreases the number of colonies formed in a colony-forming assay; and (d) a nucleic acid encoding a polypeptide encoded by a nucleic acid of (c). The defined conditions may be low stringency conditions moderate stringency conditions or high stringency conditions.

Appropriate hybridization conditions can be selected by those skilled in the art with minimal experimentation as exemplified in Ausubel et al., Current Protocols in Molecular Biology, John Wiley & Sons, 2003 (www.wiley.com/cp). Additionally, stringent conditions are described in Sambrook et al. Molecular Cloning: A Laboratory Manual, 3rd ed., Cold Spring Harbor Press, (www.molecularcloning.com). A nonlimiting example of defined conditions of low stringency is as follows. Filters containing DNA are pretreated for 6 h at 40°C. in a solution containing 35% formamide, 5x SSC, 50 mM Tris-HCl (pH 7.5), 5 mM EDTA, 0.1% PVP, 0.1% Ficoll, 1% BSA, and 500 µg/ml denatured salmon sperm DNA. Hybridizations are carried out in the same solution with the following modifications: 0.02% PVP, 0.02% Ficoll, 0.2% BSA, 100 µg/ml salmon sperm DNA, 10% (wt/vol) dextran sulfate, and 5-20x10^{6 32}P-labeled probe is used. Filters are incubated in hybridization mixture for 18-20 h at 40°C, and then washed for 1.5 h at 55°C. In a solution containing 2x SSC, 25 mM Tris-HCl (pH 7.4), 5 mM EDTA, and 0.1% SDS. The wash solution is replaced with fresh solution and incubated an additional 1.5 h at 60°C. Filters are blotted dry and exposed for autoradiography. Other conditions of low stringency well known in the art may be used (e.g.; as employed for cross-species hybridizations).

A non-limiting example of defined conditions of high stringency is as follows. Prehybridization of filters containing DNA is carried out for 8 h to overnight at 65°C in buffer composed of 6x SSC, 50 mM Tris-HCl (pH 7.5), 1 mM EDTA, 0.02% PVP, 0.02% Ficoll, 0.02% BSA, and 500 µg/ml denatured salmon sperm DNA. Filters are hybridized for 48 h at 65°C in the prehybridization mixture containing 100 µg /ml denatured salmon sperm DNA and 5-20x10⁶ cpm of ³²P-labeled probe. Washing of filters is done at 37°C for 1 h in a solution containing 2x SSC, 0.01 % PVP, 0.01% Ficoll, and 0.01% BSA. This is followed by a wash in 0.1 x SSC at 50°C for 45 minutes.

In another embodiment, the nucleic acids of the invention comprises a sequence as set out in SEQ ID NO:1. In one embodiment, the nucleic acids are identical to SEQ ID NO:1 and encode a protein that inhibits the growth of prostate cancer cells, as demonstrated, for example, in a colony-forming assay, such as the one described in Example 7. PMEPA1 nucleic acids comprising regions conserved among different species, are also provided.

In yet another example the nucleic acid comprises a contiguous stretch of at least 750 contiguous nucleotides of SEQ ID NO:1. Such contiguous fragments of SEQ ID NO:1 may also contain at least one mutation so long as the mutant sequence retains the functionality of the original sequence and the capacity to hybridize to SEQ ID NO:1 under low or high stringency conditions.

Nucleic acids encoding variants of PMEPA1 proteins are provided.

In certain examples, the presently disclosed nucleic acids further comprise a sequence encoding proteins other than PMEPA1, termed fusion partners.

The present invention also provides constructs in the form of vectors, which comprise at least one nucleic acid of the invention as described in the claims. If necessary, such vectors may additionally comprise optional elements, such as an operably linked expression control, or regulatory, elements, restriction endonuclease cleavage sites, etc.

### Polypeptides

The present invention is based in part on the discovery that PMEPA1 inhibits tumor growth. For example, as demonstrated in Example 7, expression of a recombinant PMEPA1 inhibits the growth of the prostate cancer cell lines LNCaP, PC3, DU145, and the LNCaP sublines C4, C4-2, and C4-2B in a colony-forming assay. This evidence demonstrates that PMEPA1 has a tumor suppressing function.

The PMEPA1 open reading frame encodes a 252 amino acid protein (SEQ ID NO:2) with a predicted molecular mass of 27.8 kDa, having the following sequence:

As discussed in Example 3, SEQ ID NO:2 shares 83% identity to a NEDD4 WW binding protein and contains two PY motifs, i.e., PPPY (SEQ ID NO:15) ("PY1") and PPTY (SEQ ID NO:16) ("PY2"). The PPXY motif, where X can be any amino acid, has been shown to facilitate binding with WW domain-containing proteins. We demonstrate in the Examples that PMEPA1 binds to the NEDD4 protein, which contains WW domains. NEDD4 is a ubiquitin-protein ligase (E3) that is involved in the ubiquitin-dependent proteasome-mediated protein degradation pathway.

Assays for determining whether a polypeptide, such as PMEPA1, binds to other proteins having a WW domain are well-known in the art and include strategies such as combinatorial peptide libraries, affinity chromatography, expression library screening, and yeast two-hybrid screening (Kay et al. (2000) FEBS Lett., 480:55-62; Frederick et al. (1999) Mol. Cell. Biol., 19: 2330 - 2337; Dai and Pendergast (1995) Genes Dev., 9:2569-2582; Kitamura et al. (1996) Biochem. Biophys. Res. Commun., 219:509-514; Richard et al. (1995) Mol. Cell. Biol. 15:186-197; and Sudol (1994) Oncogene 9:2145-2152).

The experimental data presented in the Examples show that PMEPA1 negatively regulates cancer cell growth. Loss of such function favors tumorigenesis or progression of existing disease. Thus, PMEPA1 may suppress tumorigenesis or cancer progression by interacting with WW domain-containing molecules. The homology of PMEPA1 to the NEDD4-binding protein and the ability of PMEPA1 to bind NEDD4 indicates that PMEPA1 may regulate protein turnover via ubiquitinylation and proteasome pathways in the cell. This mechanism is, of course, merely proposed. Moreover, it is not the only mechanism by which PMEPA1 may exert its function. The present invention is not limited to any particular mechanism of PMEPA1 activity.

PMEPA1 polypeptides or their fragments may be isolated and purified by standard methods including chromatography (e.g., ion exchange, (immuno)affinity, and sizing column chromatography), centrifugation, differential solubility, or by any other standard technique for the purification of proteins. The functional properties of the polypeptides and their fragments may be evaluated using any suitable assay, e.g., by a proliferation inhibition assay as described in Examples.

Alternatively, PMEPA1 polypeptides may be produced by recombinant DNA techniques or by chemical synthetic methods (see, e.g., Hunkapiller et al. (9984) Nature, 310:105-111) using the presently disclosed sequences of PMEPA1.

The PMEPA1 protein sequence can be characterized by a hydrophobicity analysis (Hopp et al. (1981) Proc. Natl. Acad. Sci. U.S.A., 78:3824). A hydrophilicity profile can be used to identify the hydrophobic and hydrophilic regions of the PMEPA1 protein and the corresponding regions of the gene sequence that encode such regions.

Secondary and tertiary structure analyses (Chou et al. (1974) Biochemistry, 13:222) can be carried out to identify regions of PMEPA1 that assume specific 3D structures. Other methods of structural analysis include, but are not limited to, X-ray crystallography (Engstom (1974) Biochem. Exp. Biol., 11:7-13) and computer modeling of virtual representations of the presently disclosed polypeptides (Fletterick et al. (1986) Computer Graphics and Molecular Modeling, in Current Communications in Molecular Biology, Cold Spring Harbor Laboratory, Cold Spring Harbor, NY).

### Cloning and Expression Systems

Host cells which comprise one or more nucleic acids as above, and a method for making a PMEPA1 polypeptide are also disclosed. The method comprising expressing a nucleic acid encoding PMEPA1. Expression may be achieved by culturing under appropriate conditions recombinant host cells containing the nucleic acid. Following production of a PMEPA1 polypeptide by expression, the polypeptide may be isolated and/or purified using any suitable technique.

PMEPA1 polypeptides and encoding nucleic acids and vectors may be isolated and/or purified, e.g., from their natural environment, in substantially pure or homogeneous form, or, in the case of nucleic acid, free or substantially free of nucleic acid or genes of origin other than the sequence encoding a polypeptide with the required function.

Systems for cloning and expression of a polypeptide in a variety of different host cells are well known. Suitable host cells include bacteria, mammalian cells, and yeast and baculovirus systems. Mammalian cell lines available in the art for expression of a heterologous polypeptide include Chinese hamster ovary cells, HeLa cells, baby hamster kidney cells, NS0 mouse melanoma cells, human prostate cancer cell lines PC3 and LNCaP, and many others. A common bacterial host is *E*. *coli*. For other suitable cell lines, see Fernandez et al. (1999) Gene Expression Systems, Academic Press. Any cell compatible with the present invention may be used to produce the presently disclosed polypeptides.

Suitable expression vectors can be chosen or constructed, containing appropriate regulatory sequences, including promoter sequences, terminator sequences, polyadenylation sequences, enhancer sequences, marker genes and other sequences as appropriate. Vectors may be plasmids or viral, e.g., phage, or phagemid, as appropriate. For further details see, for example, Sambrook et al., Molecular Cloning: a Laboratory Manual: 3rd ed., Cold Spring Harbor Laboratory Press (www.molecularcloning.com). Many known techniques and protocols for manipulation of nucleic acids, for example, in preparation of nucleic acid constructs, mutagenesis, sequencing, introduction of DNA into cells and gene expression, and analysis of proteins, are described in detail in Current Protocols in Molecular Biology, Ausubel et al., eds., John Wiley & Sons, 2003 (www.wiley.com/cp). Thus, also disclosed is a host cell comprising an expression vector, where the expression vector comprises a PMEPA1 nucleic acid sequence operably linked to a regulatory sequence, such as a promoter or an enhancer. The host cell maybe a eukaryotic cell, or a prokaryotic cell.

The promoter may be inducible or constitutive, and, optionally, tissue-specific. Tissue specific promoters for prostate cells include, but are not limited to, the regulatory elements of the genes encoding the rat probasin and prstatic steroid binding proteins (Allison et al. (1989) Mol. Cell Biol., 9:2254-2257; Greenberg et al. (1994) Mol. Endocrinol., 8:230-239) and regulatory elements from the genes encoding human prostate-specific antigen, prostatic acid phosphatase, DD3, and PCGEM1 (Schuur et al. (1996) J. Biol. Chem., 271:7043-7051; Zelivianski et al. (2002) Oncogene, 21:3696-3705; Bussemakers et al. (1999) Cancer Res., 59:5975-5979; Srikantan et al. (2000) Proc. Natl. Acad. Sci. U.S.A, 97:12216-12221). Other tissue-specific promoters are well-known in the art.

The vector comprising a PMEPA1 nucleic acid can be a pEGFP-C1 or pEGFP-N1 plasmid.

Thus also described is a method comprising introducing the PMEPA1 nucleic acid into a host cell. The introduction may employ any available technique. For eukaryotic cells, suitable techniques may include calcium phosphate transfection, DEAE-Dextran, electroporation, liposome-mediated transfection and transduction using retrovirus or other virus, e.g., vaccinia or, for insect cells, baculovirus. For bacterial cells, suitable techniques may include calcium chloride transformation, electroporation and transfection using bacteriophage.

The introduction may be followed by causing or allowing expression from the nucleic acid, e.g., by culturing host cells under conditions for expression of the gene.

Polymerase chain reaction (PCR) also may be used to isolate and amplify a nucleic acid of the invention as described above, for example.

Alternatively, a desired nucleic acid of the invention or a fragment thereof may be chemically synthesized using well-known techniques of polynucleotide synthesis.

### Antibodies

PMEPA1 polypeptides may be used as immunogens in order to generate antibodies that specifically bind such immunogens. Such antibodies include, but are not limited to, polyclonal, monoclonal, chimeric, single chain and Fab fragments. Antibodies to a human PMEPA1 protein can be produced. Alternatively, antibodies to a domain (e.g., a PY motif) of a PMEPA1 protein are produced. Fragments of a PMEPA1 protein identified as hydrophilic can be used as immunogens for antibody production.

Various procedures known in the art may be used for the production of polyclonal antibodies to a PMEPA1 protein. In a particular embodiment, rabbit polyclonal antibodies to an epitope of a PMEPA1 protein encoded by a sequence of SEQ ID NO:2 or a subsequence thereof, can be obtained. For the production of antibody, various host animals (including but not limited to rabbits, mice, rats, etc.) can be immunized by injection with a PMEPA1 protein. Various adjuvants may be used to increase the immunological response, depending on the host species, and including, but not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum.

For preparation of monoclonal antibodies directed toward a PMEPA1 protein, any technique, which provides for the production of antibody molecules by continuous cell lines in culture may be used. For example, the hybridoma technique originally developed by Kohler et al (1975) Nature, 256:495-497, as well as the trioma technique, the human B-cell hybridoma technique (Kozbor et al. (1983) Immunology Today, 4:72), and the EBV-hybridoma technique to produce human monoclonal antibodies (Cole et al. (1985) Monoclonal Antibodies and Cancer Therapy, Alan R. Liss, Inc., pp. 77-96). According to the invention, human antibodies may be used and can be obtained by using human hybridomas (Cote et al. (1983) Proc. Natl. Acad. Sci. U.S.A., 80:2026-2030) or by transforming human B cells with EBV virus *in vitro* (Cole et al. (1985) Monoclonal Antibodies and Cancer Therapy, Alan R. Liss, pp. 77-96). According to the invention, techniques developed for the production of chimeric antibodies (Morrison et al. (1984), Proc. Natl. Acad. Sci. U.S.A., 81:6851-6855; Neuberger et al. (1984) Nature, 312:604-608: Takeda et al. (1985) Nature, 314:452-454) by splicing the genes from a mouse antibody molecule specific for PMEPA1 together with genes from a human antibody molecule of appropriate biological activity can be used.

Techniques described for the production of single chain antibodies (U.S. Patent No. 4,946,778) can be used to produce PMEPA1-specifrc single chain antibodies. Techniques described for the construction of Fab expression libraries (Huse et al, (1989) Science, 246:1275-1281) to allow rapid and easy identification of monoclonal Fab fragments with the desired specificity for PMEPA1 proteins may be employed.

Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, the Fab fragments which can be generated by treating the antibody molecules with papain and a reducing agent, and Fv fragments, including single chain Fv (scFv) fragments.

In the production of antibodies, screening for the desired antibody can be accomplished by techniques known in the art, e.g., ELISA. For example, to select antibodies that recognize a specific domain of a PMEPA1 protein, one may assay generated hybridomas for a product which binds to a PMEPA1 fragment containing such domain.

### Variants and Analogs

The use of functionally active variants and analogs related to PMEPA1 are within the scope of the present invention These functionally active variants or analogs and at least 95% identical to SEQ ID NO:2 and are capable of exhibiting one or more activities associated with a full-length, wild-type, PMEPA1 protein. As one example, such variants or analogs that have the desired immunogenicity or antigenicity can be used, for example, in immunoassays, for immunization, for inhibition of PMEPA1 activity, etc. Variants or analogs that retain, or alternatively lack or inhibit, a desired PMEPA1 property of interest can be used as inducers, or inhibitors, respectively, of such property and its physiological correlates. These PMEPA1 properties are inhibiting cancer cell growth. Variants or analogs of PMEPA1 can be tested for the desired activity by procedures known in the art, including but not limited to, the assays described in the Examples.

By way of example, PMEPA1 variants can be made by altering PMEPA1 sequences by substitutions, additions, and/or deletions that provide for functionally equivalent molecules. DNA sequences other than SEQ ID NO:1 that encode the same amino acid sequence as a PMEPA1 gene may be used in the practice of the present invention. These include, but are not limited to, nucleotide sequences comprising PMEPA1 genes that are altered by the substitution of different codons that encode a functionally equivalent amino acid residue within the sequence, thus producing a "silent" change. Likewise, the PMEPA1 variants of the invention include those 95% identical to SEQ ID NO:2, including altered sequences in which functionally equivalent amino acid residues are substituted for residues within the sequence resulting in a silent change. For example, one or more amino acid residues within the sequence can be substituted by another amino acid of a similar polarity which acts as a functional equivalent, resulting in a "silent" alteration. Substitutes for an amino acid within the sequence may be selected from other members of the class to which the amino acid belongs. For example, the nonpolar amino acids include alanine, leucine, isoleucine, valine, proline, phenylalanine, tryptophan, and methionine. The polar neutral amino acids include glycine, serine, threonine, cysteine, tyrosine, asparagine, and glutamine. The positively charged (basic) amino acids include arginine, lysine, and histidine. The negatively charged (acidic) amino acids include aspartic acid and glutamic acid.

In one embodiment, the PMEPA1 variants contain one or more mutation and/or deletion in one or both of the PY motifs of SEQ ID No.2. These variants can be used, for example, in the treatment of hypoproliferative disorders. In addition, these variants can be used as immunogens to generate antibodies.

The PMEPA1 variants and analogs of the invention can be produced by various manipulations that can be performed at the gene or mRNA level. For example, the cloned PMEPA1 gene sequence can be modified by any of numerous strategies known in the art (Maniatis (1990) Molecular Cloning, A Laboratory Manual, 3rd ed., Cold Spring Harbor Laboratory, Cold Spring Harbor, NY (www.molecularcloning.com)). The sequence can be cleaved at appropriate sites with restriction endonuclease(s), followed by further enzymatic modification and, if desired, isolated and ligated *in vitro.* In the production of the gene encoding a variant or analog of PMEPA1, care should be taken to ensure that the modified gene remains within the same translational reading frame as PMEPA1, uninterrupted by translational stop signals, in the gene region where the desired PMEPA1 activity is encoded.

Additionally, the PMEPA1 nucleic acids can be mutated *in vitro* or *in vivo*, to create and/or destroy translation, initiation, and/or termination sequences, or to create variations in coding regions and/or form new restriction endonuclease sites or remove preexisting ones. Any technique for mutagenesis known in the art can be used, including, but not limited to, chemical mutagenesis, *in vitro* site-directed mutagenesis (Hutchinson et al. (1978) J. Biol. Chem., 253:6551), etc.

The polynucleotides can be DNA or RNA or chimeric mixtures or variants or modified versions thereof, single-stranded or double-stranded. The oligonucleotide can be modified at the base moiety, sugar moiety, or phosphate backbone. The oligonucleotide may include other appended groups such as peptides, or agents facilitating transport across the cell membrane (see, e.g., Letsinger et al. (1989) Proc. Natl. Acad. Sci. U.S.A., 86:6553-6556; Lemaitre et al. (1987) Proc. Natl. Acad. Sci., 84:648-652; PCT Pub. No. WO 88/09810) or blood-brain barrier (see, e.g., PCT Pub. No. WO 89/10134), hybridization-triggered cleavage agents (see, e.g., Krol et al. (1988) Bio/Techniques, 6:958-976) or intercalating agents (see, e.g., Zon (1988) Pharm. Res., 5:539-549).

### Therapeutic Uses

The invention provides for treatment or prevention of prostrate cancer by administration of a therapeutic compound (termed herein "therapeutic"). "Therapeutics" include but are not limited to: PMEPA1 proteins as disclosed in the claims; nucleic acids encoding the PMEPA1 proteins as disclosed in the claims. The therapeutic effect of PMEPA1 therapy can be monitored by measuring parameters that are routinely used to measure disease and/or cancer progression. For example, the therapeutic effect of PMEPA1 in prostate cancer can be determined by measuring the serum levels of androgen-regulated genes, such as prostate specific antigen (PSA), which is routinely used to monitor prostate cancer progression, particularly following prostate surgery.

### Hyperproliferative Disorders

Disorders involving hyperproliferation of cells are treated or prevented by administration of a therapeutic that promotes (activates) PMEPA1 function. Disorders in which cell proliferation is deficient or is desired are treated or prevented by administration of a therapeutic that antagonizes (inhibits) PMEPA1 function. The above is described in detail below.

Therapeutics which are useful for treatment of a disorder may be selected by testing for biological activity in promoting the survival or differentiation of cells. For example, in a specific embodiment relating to prostrate cancer therapy, a therapeutic decreases proliferation of tumor cells. These effects can be measured as described in the Examples or using any other method standard in the art.

Examples of such a therapeutic that promotes (i.e., increases of supplies) PMEPA1 function include, PMEPA1 proteins, including variants that are functionally active, particularly that are active in inhibiting cell proliferation (e g., as demonstrated in *in vitro* assays or in animal models), and nucleic acids encoding the PMEPA1 proteins (e.g., for use in gene therapy). Other therapeutics that can be used but which are not within the scope of the invention, e.g., PMEPA1 agonists, can be identified using *in vitro* assays or animal models, examples of which are described below.

In specific examples, therapeutics that promote PMEPA1 function are administered therapeutically (including prophylactically) in: (1) diseases or disorders involving an absence or decreased (relative to normal or desired) level of PMEPA1 protein or function, for example, PMEPA1 protein is lacking, genetically defective, biologically inactive or underactive, or underexpressed; or (2) diseases or disorders where *in vitro* or *in vivo* assays indicate the utility of PMEPA1 agonist administration. The absence or decreased level of PMEPA1 protein or function can be readily detected, e.g., by obtaining a biological sample from a patient, e.g., a tissue sample (e.g., from biopsy tissue), a blood sample, or a urine sample and assaying it *in vitro* for mRNA or protein levels, structure and/or activity of the expressed PMEPA1 mRNA or protein. Many methods standard in the art can be employed, including but not limited to, kinase assays, immunoassays to detect and/or visualize PMEPA1 protein (e.g., Western blot, immunoprecipitation followed by SDS-PAGE, immunocytochemistry, etc.) and/or hybridization assays to detect PMEPA1 expression by detecting and/or visualizing PMEPA1 mRNA (e.g., Northern assays, dot blots, in situ hybridization, RT-PCR, etc.).

Diseases and disorders involving hyperproliferation that can be treated or prevented include malignancies of the prostate.

Malignancy or dysproliferative changes (such as metaplasias and dysplasias), or hyperproliferative disorders, are treated or prevented in the prostate.

The therapeutics that promote PMEPA1 activity can also be administered to treat premalignant conditions and to prevent progression to a neoplastic or malignant state, including but not limited to those disorders described herein. Such prophylactic or therapeutic use is indicated in conditions known or suspected of preceding progression to neoplasia or cancer, in particular, where non-neoplastic cell growth consisting of hyperplasia, metaplasia, or most particularly, dysplasia has occurred (for review of such abnormal growth conditions, see Robbins et al. (1976) Basic Pathology, 2nd ed., W.B. Saunders Co., PA, pp. 68-79). Hyperplasia is a form of controlled cell proliferation involving an increase in cell number in a tissue or organ, without significant alteration in structure or function. For example, endometrial hyperplasia often precedes endometrial cancer. Metaplasia is a form of controlled cell growth in which one type of adult or fully differentiated cell substitutes for another type of adult cell. Metaplasia can occur in epithelial or connective tissue cells. Atypical metaplasia involves a somewhat disorderly metaplastic epithelium. Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exists chronic irritation or inflammation, and is often found in the cervix, respiratory passages, oral cavity, and gall bladder.

Alternatively or in addition to the presence of abnormal cell growth characterized as hyperplasia, metaplasia, or dysplasia, the presence of one or more characteristics of a transformed phenotype, or of a malignant phenotype, displayed *in vivo* or displayed *in vitro* by a cell sample from a patient, can indicate the desirability of prophylactic/therapeutic administration of a therapeutic that promotes PMEPA1 function. Such characteristics of a transformed phenotype include morphology changes, looser substratum attachment, loss of contact inhibition, loss of anchorage dependence, protease release, increased sugar transport, decreased serum requirement, expression of fetal antigens, etc.

A patient who exhibits one or more of the following predisposing factors for malignancy can be treated by administration of an effective amount of a therapeutic: a chromosomal translocation associated with a malignancy familial polyposis or Gardner's syndrome (possible forerunners of colon cancer), benign monoclonal gammopathy (a possible forerunner of multiple myeloma), and a first degree kinship with persons having a cancer or precancerous disease showing a Mendelian (genetic) inheritance pattern (e.g., familial polyposis of the colon, Gardner's syndrome, hereditary exostosis, polyendocrine adenomatosis, medullary thyroid carcinoma with amyloid production and pheochromocytoma, Peutz-Jeghers syndrome, neurofibromatosis of Von Recklinghausen, retinoblastoma, carotid body tumor, cutaneous melanocarcinoma, intraocular melanocarcinoma, xeroderma pigmentosum, ataxia telangiectasia, Chediak-Higashi syndrome, albinism, Fanconi's aplastic anemia, and Bloom's syndrome; see Robbins et al. (1976) Basic Pathology, 2nd ed., W.B. Saunders Co., PA, pp. 112-113, etc.). A therapeutic may also be administered to a human patient to prevent progression to breast, colon, lung, pancreatic, or uterine cancer, or melanoma or sarcoma.

### Hypoproliferative Disorders

### These are discussed by way of comparison only and do not fall within the scope of the invention.

Diseases and disorders involving a deficiency in cell proliferation (growth) or in which cell proliferation is otherwise desirable for treatment or prevention, are treated or prevented by administration of a therapeutic that antagonizes (inhibits) PMEPA1 function (in particular, PMEPA1-mediated inhibition of cell proliferation). Therapeutics that can be used include, but are not limited to, anti-PMEPA1 antibodies (and fragments and variants thereof containing the binding region thereof), PMEPA1 variants or analogs that antagonize PMEPA1 (including variants that contain at least one mutation and/or deletion in at least one of the PMEPA1 PY motifs), PMEPA1 antisense nucleic acids, and PMEPA1 nucleic acids that are dysfunctional (e.g., due to a heterologous (non-PMEPA1 sequence) insertion within the PMEPA1 coding sequence) that are used to knock out endogenous PMEPA1 function by homologous recombination (see, e.g., Capecchi (1989) Science, 244:1288-1292).

For example a nucleic acid containing a portion of a PMEPA1 gene in which PMEPA1 sequences flank a different gene sequence is used as a PMEPA1 antagonist to promote PMEPA1 inactivation by homologous recombination (see also Koller et al. (1989) Proc. Natl. Acad. Sci. U.S.A., 86:8932-8935; Zijlstra et al. (1989) Nature, 342:435-438). Other therapeutics that inhibit PMEPA1 function can be identified by use of known convenient *in vitro* assays, e.g., based on their ability to inhibit binding of PMEPA1 to another protein (e.g., a WW domain containing protein), or inhibit any known PMEPA1 function, as assayed *in vitro,* although genetic assays (e.g., in Drosophila) may also be employed. Suitable *in vitro* or *in vivo* assays, may be utilized to determine the effect of a specific therapeutic and whether its administration is indicated for treatment of the affected tissue.

Therapeutics that inhibit PMEPA1 function are administered therapeutically (including prophylactically) in: (1) diseases or disorders involving an increased level of PMEPA1 protein or function, for example, PMEPA1 protein is overactive or overexpressed; or (2) diseases or disorders wherein *in vitro* or *in vivo* assays indicate the utility of PMEPA1 antagonist administration. The increased levels in PMEPA1 protein or function can be readily detected, e.g., by quantifying protein and/or mRNA, by obtaining a patient tissue sample (e.g., from biopsy tissue) and assaying it *in vitro* for mRNA or protein levels, structure and/or activity of the expressed PMEPA1 mRNA or protein. Many methods standard in the art can be thus employed, including, but not limited to, kinase assays, immunoassays to detect and/or visualize PMEPA1 protein (e.g., Western blot, immunoprecipitation followed by SDS-PAGE, immunocytochemistry, etc.) and/or hybridization assays to detect PMEPA1 expression by detecting and/or visualizing respectively PMEPA1 mRNA (e.g., Northern assays, dot blots, in situ hybridization, etc.).

Diseases and disorders involving a deficiency in cell proliferation or in which cell proliferation is desired for treatment or prevention, and that can be treated or prevented by inhibiting PMEPA1 function, include, but are not limited to, degenerative disorders, growth deficiencies, hypoproliferative disorders, physical trauma, lesions, and wounds. For example, PMEPA1 antagonism can be used to promote wound healing, or regeneration of lesions or injury, etc.

Lesions which may be treated include, but are not limited to, the following lesions: (i) traumatic lesions, including lesions caused by physical injury or associated with surgery; (ii) ischemic lesions, in which a lack of oxygen results in cell injury or death, e.g., myocardial or cerebral infarction or ischemia or spinal cord infarction or ischemia; (iii) malignant lesions, in which cells are destroyed or injured by malignant tissue; (iv) infectious lesions, in which tissue is destroyed or injured as a result of infection, for example, by an abscess or associated with infection by human immunodeficiency virus, herpes zoster, or herpes simplex virus, or with Lyme disease, tuberculosis or syphilis; (v) degenerative lesions, in which tissue is, destroyed or injured as a result of a degenerative process, including, but not limited to, nervous system degeneration, associated with Parkinson's disease, Alzheimer's disease, Huntington's chorea or amyotrophic lateral sclerosis; (vi) lesions associated with nutritional diseases, or disorders, in which tissue is destroyed or injured by a nutritional disorder or disorder of metabolism including but not limited to, vitamin B12 deficiency, folic acid deficiency, Wernicke disease, tobacco-alcohol amblyopia, Marchiafava-Bignami disease (primary degeneration of the corpus callosum) and alcoholic cerebellar degeneration; (vii) lesions associated with systemic diseases, including, but not limited to, diabetes or systemic lupus erythematosus; (viii) lesions caused by toxic substances including alcohol, lead, or other toxins; and (ix) demyelinated lesions of the nervous system in which a portion of the nervous system is destroyed or injured by a demyelinating disease including, but not limited to, multiple sclerosis, human immunodeficiency virus-associated myelopathy, transverse myelopathy, progressive multifocal leukoencephalopathy, and central pontine myelinolysis.

PMEPA1 function may be inhibited by use of PMEPA1 antisense nucleic acids. The therapeutic or prophylactic use of nucleic acids of at least about 10, 15. 100, 200, 500, 600, 700 or 750 contiguous nucleotides in antisense to any of the PMEPA1 nucleotides is described herein. For example, the PMEPA1 antisense nucleic acid comprises at least about 10, 15, 100, 200, 500, 600, 700, or 750 contiguous nucleotides in antisense orientation to SEQ ID NO:1. A PMEPA1 "antisense" nucleic acid as used herein refers to a nucleic acid capable of hybridizing to a portion of a PMEPA1 RNA (preferably mRNA) by virtue of some sequence complementarity. The antisense nucleic acid may be complementary to a coding and/or noncoding region of a PMEPA1 mRNA. Such antisense nucleic acids have utility as therapeutics that inhibit PMEPA1 function, and can be used in the treatment or prevention of disorders as described herein.

The antisense nucleic acids can be oligonucleotides that are double-stranded or single-stranded, RNA or DNA or a modification or variant thereof, which can be directly administered to a cell, or which can be produced intracellularly by transcription of exogenously, introduced coding sequences.

### Gene Therapy

In a specific embodiment, nucleic acids comprising a sequence encoding a PMEPA1 protein are administered to promote PMEPA1 function, by way of gene therapy. Gene therapy refers to therapy performed by the administration of a nucleic acid to a subject. In this embodiment of the invention, the nucleic acid produces its encoded protein that mediates a therapeutic effect by promoting PMEPA1 function.

Any of the methods for gene therapy available in the art can be used according to the present invention. Exemplary methods are described bellow.

For specific protocols, see Morgan (2001) Gene Therapy Protocols, 2nd ed., Humana Press. For general reviews of the methods of gene therapy, see Goldspiel et al. (1993) Clinical Pharmacy, 12:488-505; Wu et al. (1991) Biotherapy, 3:87-95; Tolstoshev (1993) Ann. Rev. Pharmacol. Toxicol., 32:573-596; Mulligan (1993) Science, 260:926-932; and Morgan et al. (1993) Ann. Rev. Biochem., 62:191-217; May (1993) TIBTECH, 11(5):155-215). Methods commonly known in the art of recombinant DNA technology which can be used are described in Current Protocols in Molecular Biology, Ausubel et al., eds., John Wiley & Sons 2003 (www.wiley.com/cp), NY; and Kriegler (1990) Gene Transfer and Expression, A Laboratory Manual, Stockton Press, NY.

In one embodiment, the therapeutic comprises a PMEPA1 nucleic acid that is part of an expression vector that expresses a PMEPA1 protein or chimeric protein thereof in a suitable host. In particular, such a nucleic acid has a regulatory sequence, such as a promoter, operably linked to the PMEPA1 coding region, said promoter being inducible or constitutive, and, optionally, tissue-specific. In another particular embodiment, a nucleic acid molecule is used in which the PMEPA1 coding sequences and any other desired sequences are flanked by regions that promote homologous recombination at a desired site in the genome, thus providing for intrachromosomal expression of the PMEPA1 nucleic acid (Koller et al. (1989) Proc. Natl. Acad. Sci. U.S.A., 86:8932-8935; Zijlstra et al. (1989) Nature, 342:435-438).

In a specific embodiment, the nucleic acid to be introduced for purposes of gene therapy comprises an inducible promoter operably linked to the coding region, such that expression of the nucleic acid is controllable by the appropriate inducer of transcription.

Delivery of the nucleic acid into a patient may be either direct, in which case the patient is directly exposed to the nucleic acid or nucleic acid-carrying vector, or indirect, in which case, cells are first transformed with the nucleic acid *in vitro*, then transplanted into the patient. These two approaches are known, respectively, as *in vivo* or *ex vivo* gene therapy.

In a specific embodiment, the nucleic acid is directly administered *in vivo*, where it is expressed to produce the encoded product. This can be accomplished by any of numerous methods known in the art, e.g., by constructing it as part of an appropriate nucleic acid expression vector and administering it so that it becomes intracellular, e.g., by infection using a defective or attenuated retroviral or other viral vector (see U.S. Patent No. 4,980,286), or by direct injection of naked DNA, or by use of microparticle bombardment (e.g., a gene gun; Biolistic, DuPont), or coating with lipids or cell-surface receptors or transfecting agents, encapsulation in liposomes, microparticles, or microcapsules, or by administering it in linkage to a peptide which is known to enter the nucleus, by administering it in linkage to a ligand subject to receptor-mediated endocytosis (see, e.g., Wu et al. (1987) J. Biol. Chem., 262:4429-4432). In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted *in vivo* for cell-specific uptake and expression, by targeting a specific receptor (see, e.g., PCT Pubs. WO 92/06180; WO 92/22635; WO92/20316; WO93/14188; WO 93/20221). Alternatively, the nucleic acid can be introduced intracellularly and incorporated within host cell DNA for expression, by homologous recombination (Koller et al. (1989) Proc. Natl. Acad. Sci. U.S.A., 86:8932-8935; Zijlstra et al. (1989) Nature, 342:435-438).

In a specific embodiment, a viral vector that contains a PMEPA1 nucleic acid is used. For example, a retroviral vector can be used (see, Miller et al. (1993) Meth. Enzymol., 217:581-599). These retroviral vectors have been modified to delete retroviral sequences that are not necessary for packaging of the viral genome and integration into host cell DNA. The PMEPA1 nucleic acid to be used in gene therapy is cloned into the vector, which facilitates delivery of the gene into a patient. More detail about retroviral vectors can be found in Boesen et al. (1994) Biotherapy, 6:291-302, which describes the use of a retroviral vector to deliver the MDRL gene to hematopoietic stem cells in order to make the stem cells more resistant to chemotherapy. Other references illustrating the use of retroviral vectors in gene therapy are: Clowes et al. (1994) J. Clin. Invest., 93:644-651; Kiem et al. (1994) Blood, 83:1457-1473; Salmons et al. (1993) Hum. Gene Ther., 4:129-141; and Grossman et al. (1993) Curr. Opin. Gen. Devel., 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky et al. (1993, Curr. Opin. Gen. Devel., 3:499-503) present a review of adenovirus-based gene therapy. Bout et al. (1994, Hum. Gene Ther., 5:3-10) demonstrated the use of adenovirus vectors to transfer genes to the respiratory epithelia of rhesus monkeys. Other instances of the use of adenoviruses in gene therapy can be found in Rosenfeld et al. (1991) Science, 252:431-434; Rosenfeld et al. (1992) Cell, 68:143-155; and Mastrangeli et al. (1993) J. Clin. Invest, 91:225-234.

Adeno-associated virus (AAV) has also been proposed for use in gene therapy (Walsh et al. (1993) Proc. Soc. Exp. Biol. Med., 204:289-300).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this approach the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, e.g., Loeffler et al. (1993) Meth. Enzymol., 217:599-618; Cohen et al. (1993) Meth. Enzymol., 217:618-644; Cline (1985) Pharmac. Ther., 29:69-92) and may be used provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a patient by various methods known in the art. In one example, epithelial cells are injected, e.g., subcutaneously. In another example recombinant skin cells may be applied as a skin graft onto the patient. Recombinant blood cells (e.g., hematopoietic stem or progenitor cells) may be administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include, but are not limited to, epithelial cells, endothelial cells, keratinocytes, fibroblasts, muscle cells, hepatocytes, T lymphocytes, B lymphocytes, monocytes, macrophages, neutrophils, eosinophils, megakaryocytes, granulocytes; various stem or progenitor cells, in particular hematopoietic stem or progenitor cells, e.g., as obtained from bone marrow, umbilical cord blood, peripheral blood, fetal liver, etc. In certain embodiments, the cells used for gene therapy are autologous to the patient.

In one example, a PMEPA1 nucleic acid is introduced into the cells such that it is expressible by the cells or their progeny, and the recombinant cells are then administered *in vivo* for therapeutic effect. In a specific example, stem or progenitor cells are used. Any stem and/or progenitor cells which can be isolated and maintained *in vitro* can potentially be used in accordance with this example. Such stem cells include, but are not limited to, hematopoietic stem cells (HSC), stem cells of epithelial tissues such as the skin and the lining of the gut, embryonic heart muscle cells, liver stem cells (PCT Pub. WO 94/08598), and neural stem cells (Stemple et al. (1992) Cell, 71:973-985).

Epithelial stem cells (ESCs) or keratinocytes can be obtained from tissues such as the skin and the lining of the gut by known procedures (Rheinwald (1980) Meth. Cell Bio., 21A:229). In stratified epithelial tissue such as the skin, renewal occurs by mitosis of stem cells within the germinal layer, the layer closest to the basal lamina. Stem cells within the lining of the gut provide for a rapid renewal rate of this tissue. ESCs or keratinocytes obtained from the skin or lining of the gut of a patient or donor can be grown in tissue culture (Rheinwald (1980) Meth. Cell Bio., 21A:229; Pittelkow et al. (1986) Mayo Clinic. Proc., 61:771). If the ESCs are provided by a donor, a method for suppression of host versus graft reactivity (e.g., irradiation, drug or antibody administration to promote moderate immunosuppression) can also be used.

With respect to hematopoietic stem cells (HSC), any technique which provides for the isolation, propagation, and maintenance *in vitro* of HSC can be used. Techniques by which this may be accomplished include (a) the isolation and establishment of HSC cultures from bone marrow cells isolated from the future host, or a donor, or (b) the use of previously established long-term HSC cultures, which may be allogeneic or xenogeneic. Non-autologous HSC may be used in conjunction with a method of suppressing transplantation immune reactions of the future host/patient. In a particular example, human bone marrow cells can be obtained from the posterior iliac crest by needle aspiration (see, e.g., Kodo et al. (1984) J. Clin. Invest., 73:1377-1384). In one example the HSCs can be made highly enriched, isolated, or in substantially pure form. This enrichment can be accomplished before, during, or after long-term culturing, and can be done by any techniques known in the art. Long-term cultures of bone marrow cells can be established and maintained by using, for example, modified Dexter cell culture techniques (Dexter et al. (1977) J. Cell Physiol., 91:335) or Witlock-Witte culture techniques (Witlock et al. (1982) Proc. Natl. Acad. Sci. U.S.A., 79:3608-3612).

### Diagnosis

PMEPA1 proteins and PMEPA1 nucleic acids (and their complementary and homologous sequences) and antibodies thereto, including anti-PMEPA1 antibodies, have uses in diagnostics. Such molecules can be used in assays, such as immunoassays, to detect, prognose, diagnose, or monitor various conditions, diseases, and disorders affecting PMEPA1 expression, or monitor the treatment thereof. In particular, such an immunoassay is carried out by a method comprising contacting a sample derived from a patient with an anti-PMEPA1 antibody under conditions such that specific binding can occur, and detecting or measuring the amount of any specific binding by the antibody. In one embodiment, such binding of antibody, in tissue sections, can be used to detect aberrant PMEPA1 localization or aberrant (e.g., low or absent) levels of PMEPA1. In a specific embodiment, antibody to PMEPA1 can be used to assay in a patient tissue or serum sample for the presence of PMEPA1 where an aberrant level of PMEPA1 is an indication of a diseased condition.

The immunoassays which can be used include, but are not limited to, competitive and non-competitive assay systems using techniques such as Western blots, radioimmunoassays, ELISA, immunoprecipitation assays, immunodiffusion assays, agglutination assays, complement-fixation assays, immunoradiometric assays, fluorescent immunoassays, protein A immunoassays, to name but a few.

PMEPA1 genes and related nucleic acid sequences and subsequences, including complementary sequences, can also be used in hybridization assays. PMEPA1 nucleic acid sequences, or subsequences thereof comprising about at least 8 nucleotides, can be used as hybridization probes. Hybridization assays can be used to detect, prognose, diagnose, or monitor conditions, disorders, or disease states associated with aberrant changes in PMEPA1 expression and/or activity as described above. In particular, such a hybridization assay is carried out by a method comprising contacting a sample containing nucleic acid with a nucleic acid probe capable of hybridizing to PMEPA1 nucleic acid, under conditions such that hybridization can occur and detecting or measuring the degree of the resulting hybridization. As described herein, PCR/RT-PCR can be used to detect the presence of the PMEPA1 gene and/or level of its mRNA expression.

In specific examples diseases and disorders involving hyperproliferation of cells can be diagnosed and/or prgonosed, or a predisposition to develop such disorders can be predicted, by detecting decreased levels of the PMEPA1 protein, PMEPA1 nucleic acid, or PMEPA1 functional activity (e.g., inhibiting cancer cell growth, inhibiting androgen receptor expression, etc.), or by detecting mutations in PMEPA1 RNA, DNA, or protein (e.g., translocations in PMEPA1 nucleic acids, truncations in the PMEPA1 gene or protein, changes in nucleotide or amino acid sequence relative to wild-type PMEPA1) that cause decreased expression or activity of PMEPA1. By way of example, levels of PMEPA1 protein can be detected by immunoassay, levels of PMEPA1 mRNA can be detected by hybridization assays (e.g., Northern blots, dot blots, RT-PCR), PMEPA1 binding to a WW domain-containing protein can be done by binding assays commonly known in the art, translocations and point mutations in PMEPA1 nucleic acids can be detected by Southern blotting. RFLP analysis, PCR, sequencing of the PMEPA1 genomic DNA or cDNA obtained from the patient, etc.

In one embodiment, levels of the PMEPA1 mRNA or protein in a subject sample are detected or measured, in which decreased levels, relative to a matched, normal tissue sample, indicate that the subject has, or has a predisposition to developing, a malignancy or hyperproliferative disorder of the prostate. In one embodiment, a method of diagnosing cancer comprises determining in a biological sample obtained from a subject (including but not limited to, a tissue sample (e.g., from biopsy tissue), a blood sample, or a urine sample), the expression level of a PMEPA1 gene, PMEPA1 polypeptide, and/or PMEPA1 activity, and diagnosing or prognosing cancer (e.g., prostate cancer) in said subject. In further embodiments, the expression level of the PMEPA1 gene and/or PMEPA1 activity is determined by Southern blotting, Northern blotting, Western blotting, ELISA, RT-PCR or other techniques as described herein or known in the art.

### Screening for Modulatory Compounds

### Methods of screening for modulatory compounds are discussed, but do not fall within the scope of the current invention.

PMEPA1 Nucleic acids and proteins also have uses in screening assays to detect molecules that specifically bind to PMEPA1 nucleic acids or proteins, or that can modulate expression of PMEPA1 nucleic acids or proteins, and thus have potential use as agonists or antagonists of PMEPA1, in particular, molecules that are capable of modulating cell proliferation. Such assays can be performed to screen for molecules with potential utility as anti-cancer drugs or lead compounds for further drug development. For example, recombinant cells expressing PMEPA1 nucleic acids can be used to recombinantly produce PMEPA1 proteins to screen for molecules that bind to the PMEPA1 protein. Molecules (e.g., putative binding partners of PMEPA1) are contacted with the PMEPA1 protein (or fragment thereof) under conditions suitable for conducive to binding, and then molecules that specifically bind to the PMEPA1 protein are identified. Similar methods can be used to screen for molecules that bind to PMEPA1 nucleic acids. Methods that can be used to carry out the foregoing are commonly known in the art.

By way of example, diversity libraries such as random or combinatorial peptide or nonpeptide libraries can be screened for molecules that specifically bind to PMEPA1. Many libraries are known in the art that can be used, e.g., chemically synthesized libraries, recombinant (e.g., phage display libraries), and *in vitro* translation-based libraries.

Examples of chemically synthesized libraries are described in Fodor et al. (1991) Science, 251:767-773; Houghten et al. (1991) Nature, 354:84-86; Lam et al. (1991) Nature, 354:82-84; Medynski (1994) Bio/Technology, 12:709-710; Gallop et al. (1994) J. Medicinal Chemistry, 37(9):1233-1251; Ohlmeyer et al. (1993) Proc. Natl. Acad. Sci. U.S.A., 90:10922-10926; Erb et al. (1994) Proc. Natl. Acad. Sci. U.S.A., 91:11422-11426; Houghten et al. (1992) Biotechniques, 13:412; Jayawickreme et al. (1994) Proc. Natl. Acad. Sci. U.S.A., 91:1614-1618; Salmon et al. (1993) Proc. Natl. Acad. Sci. U.S.A., 90:11708-11712; PCT Pub. No. WO 93/20242; and Brenner et al. (1992) Proc. Natl. Acad. Sci. U.S.A., 89:5381-5383.

Examples of phage display libraries are described in Scott et al. (1990) Science, 249:386-390; Devlin et al. (1990) Science, 249:404-406; Christian et al. (1992) J. Mol. Biol., 227:711-718; Lenstra (1992) J. Immunol. Meth., 152:149-157; Kay et al. (1993) Gene, 128:59-65; and PCT Pub. No. WO 94/18318.

*In vitro* translation-based libraries include but are not limited to those described in PCT Pub. No. WO 91/05058; and Mattheakis et al. (1994) Proc. Natl. Acad. Sci. U.S.A., 91:9022-9026.

Examples of nonpeptide libraries include, but are not limited to, a benzodiazepine library (see e.g., Bunin et al. (1994) Prcc. Natl. Acad. Sci. U.S.A., 91:4708-4712) can be adapted for use. Peptoid libraries (Simon et al. (1992) Proc. Natl. Acad. Sci. U.S.A., 89:9367-9371) can also be used. Another example of a library that can be used, in which the amide functionalities in peptides have been permethylated to generate a chemically transformed combinatorial library, is described by Ostresh et al. (1994) Proc. Natl. Acad. Sci. U.S.A., 91:11138-11142).

Screening the libraries can be accomplished by any of a variety of commonly known methods. See, e.g., the following references, which disclose screening of peptide libraries: Parmley et al. (1989) Adv. Exp. Med. Biol., 251:215-218; Scott et al. (1990) Science, 249:386-390; Fowlkes et al. (1992) BioTechniques, 13:422-427; Oldenburg et al. (1992) Proc. Natl. Acad. Sci. U.S.A., 89:5393-5397; Yu et al. (1994) Cell, 76:933-945; Staudt et al. (1988) Science, 241:577-580; Bock et al. (1992) Nature, 355:564-566; Tuerk et al. (1992) Proc. Natl. Acad. Sci. U.S.A., 89:6988-6992; Ellington et al. (1992) Nature, 355:850-852; U.S. Patent No. 5,096,815, U.S. Patent No. 5,223,409, and U.S. Patent No. 5,198,346, all to Ladner et al.; Rebar et al. (1993) Science, 263:671-673; and PCT Pub. No. WO 94/18318.

For example, screening can be carried out by contacting the library members with a PMEPA1 protein (or nucleic acid) immobilized on a solid phase and harvesting those library members that bind to the PMEPA1 protein or nucleic acid. Examples of such screening methods, termed "panning" techniques are described by way of example in Parmley et al. (1988) Gene, 73:305-318; Fowlkes et al. (1992) BioTechniques, 13:422-427; PCT Publ. No. WO 94/18318; and references cited herein above.

In a further example, a two-hybrid system for selecting interacting proteins in yeast (Fields et al (1989) Nature, 340:245-246; Chien et al. (1991) Proc. Natl. Acad. Sci. U.S.A., 88:9578-9582) can be used to identify molecules that specifically bind to a PMEPA1 protein. In yet another example, the method of screening for modulatory compound comprises contacting a cell with a test compound and determining the change in expression level of PMEPA1 gene or PMEPA1 activity.

In addition, an animal model as described below can be used as a model system in order to detect genes that interacdt with PMEPA1.

### Animal Models

### Animal models are described by way of example, but do not fall within the scope of the invention.

Such an animal can be initially produced by promoting homologous recombination between a PMEPA1 gene in its chromosome and an exogenous PMEPA1 gene that has been rendered biologically inactive (preferably by insertion of a heterologous sequence, e.g., an antibiotic resistance gene). See, generally, Transgenic Mouse Methods and Protocols, Hofker et al., eds., Humana Press, 2002. This homologous recombination may be carried out by transforming embryo-derived stem (ES) cells with a vector containing the instertionally inactivated PMEPA1 gene, such that homologous recombination occurs, followed by injecting the ES cells into a blastocyst, and implanting the the blastocyst into a foster mother, followed by the birth of the chimeric animal ("knockout animal") in which a PMEPA1 gene has been inactivated (see Gene Knockout Protocols, Tymm et al., eds., Humana Press, 2001. Capecchi (1989) Science, 244:1288-1292). The chimeric animal can be mice, hamsters, sheep, pigs, cattle, etc., and ar preferably non-human mammals.

Such knockout animals are expected to develop or be predisposed to developing diseases or disorders involving cell hyperproliferation (e.g., malignancy) and thus can have use as animal models of such diseases and disorders, e.g., to screen for or test molecules (e.g., potential anti-cancer therapeutics) for the ability to inhibit hyperproliferation (e.g., tumorigenesis) any thus treat or prevent such diseases or disorders.

Alternatively transgenic animals that have incorporated and express a functional PMEPA1 gene can have use as animal models of diseases and disorders involving deficiencies in cell proliferation or in which cell proliferation is desired. Such animals can be used to screen for or test molecules for the ability to promote proliferation and thus treat or prevent such diseases and disorders.

The following examples further illustrate certain aspects of the invention.

### EXAMPLE 1

### Cell Culture and Androgen Stimulation

LNCaP cells (American Type Culture Collection, Rockville, MD) are an art-recognized model of human prostate cancer [Hsieh et al., Cancer Res., 53: 2852-7, 1993; Thalmann et al., Cancer Res., 54: 2577-81, 1994; Wu et al., Int. J. Cancer, 77: 887-94, 1998]. LNCaP cells were maintained in RPMI 1640 (Life Technologies, Inc., Gaithersburg, MD) supplemented with 10% fetal bovine serum (FBS, Life Technologies, Inc., Gaithersburg, MD) and experiments were performed on cells between passages 20 and 30.

For the studies of androgen regulation, charcoal/dextran stripped androgen-free FBS (cFBS, Gemini Bio-Products, Inc., Calabasas, CA) was used. LNCaP cells were cultured first in RPMI 1640 with 10% cFBS for 5 days and then stimulated with 10⁻⁸ M of nonmetabolizable androgen analog, R1881 (DUPONT, Boston, MA) for 24 hours. LNCaP cells identically treated but without R1881 treatment served as control. Cells were harvested at indicated time and polyA+ RNA was double-selected with Fast Track kit (Invitrogene). The quality of polyA+ was checked by Northern hybridization analysis.

Androgen (R1881) supplementation of the LNCaP cell culture media lacking androgen caused induction of both about 2.7 base pair and about 5.0 base pair RNA species of PMEPA1 in LNCaP cells in a dose and time dependent fashion (Fig. 1). Androgen depletion of LNCaP cells resulted in decreased expression of PMEPA1. Basal level of PMEPA1 expression was detected in normal prostatic epithelial cell cultures and androgen-dependent LNCaP cells cultured in regular medium. In comparison to androgen dependent LNCaP cells, androgen-independent prostate cancer cell lines DU145 and PC3 expressed very low to undetectable levels of PMEPA1 (data not shown).

RT-PCR analysis of RNA from LNCaP cells with four pairs of primers covering different regions of PMEPA1 protein coding region revealed expected size of bands from PCR reactions, suggesting that two mRNA species on northern blot have identical sequences in the protein coding region and may exhibit differences in 5' and/or 3' non-coding regions.

### EXAMPLE 2

### Analysis of PMEPA1 Tissue Expression

Analysis of multiple northern blots containing 23 human normal tissues revealed the highest level of PMEPA1 expression in prostate tissue. Although other tissues expressed PMEPA1, their relative expression was significantly lower as compared to prostate (Fig. 2). *In situ* RNA hybridization analysis of PMEPA1 expression in prostate tissues revealed abundant expression in the glandular epithelial compartment as compared to the stromal cells. However, both normal and tumor cells in tissue sections of primary tumor tissues revealed similar levels of expression.

### EXAMPLE 3

### Structural Features of the PMEPA1 Gene.

Analysis of a 1,141 base pair PMEPA1 cDNA sequence revealed an open reading frame of 759 nucleotides (SEQ ID NO:1) that encodes a 252 amino acid protein (SEQ ID NO:2). A protein motif search using ProfileScan (http://www.ch.embnet.org/cgi-bin/TMPRED) indicated the existence of a type Ib transmembrane domain between amino acid residues 9 to 25 of the PMEPA1 sequence. In addition, the motif search revealed two PY motifs in the PMEPA1 protein sequence, PPPY (SEQ ID NO:15) ("PY1") and PPTY (SEQ ID NO:16) ("PY2"). The PY motif is a proline-rich peptide sequence with a consensus PPXY sequence (where X represents any amino acid) that can bind to proteins with WW domains [Jolliffe et al., Biochem. J., 351: 557-565, 2000; Harvey et al., Trends Cell Biol., 9: 166-169, 1999; Hicke, Cell, 106: 527-530, 2001; Kumar et al., Biochem. Biophys. Res. Commun., 185: 1155-1161, 1992; Kumar et al., Genomics, 40: 435-443, 1997; Sudol, Trends Biochem. Sci., 21: 161-163, 1996; Harvey et al., J. Biol. Chem., 277: 9307-9317, 2002; and Brunschwig et al., Cancer Res., 63: 1568-1575, 2003].

A protein sequence homology search revealed that PMEPA1 has an 83% sequence identity with a mouse NEDD4 WW binding protein 4 ("N4WBP4," Accession number AK008976) (4), as shown below in Table 2. In Table 2, the + denotes a conservative substitution, and the PY motifs are underlined.

**TABLE 2**

| | |
|---|---|
| Human PMEPA1: | |
| Mouse N4WBP4: | 18 ITELEFQIVVIVVVMMVMVVMITCLLSHYKLSARSFISRHQARRRDDGLSSEGCLWPS 77 |
| Human PMEPA1: | |
| Mouse N4WBP4: | 78 ESTVSG-GMPEQVYAPPRPTDLAVPPFIQRS---RFQPTYPYLQHEIALPPTISLSDG 133 |
| Human PMEPA1: | |
| Mouse N4WBP4: | 134 EEPPPYQGPCTLQLRDPEQQLELNRESVRAPPNRTIFDSDLIDSTMLGGPCPPSSNSGIS 193 |
| Human PMEPA1: | |
| Mouse N4WBP4: | 194 ATCYSSGGRMEGPPPTYSEVIGHYPGSSFQHQQSNGPSSLLEGTRLHHSHIAPLE----- 248 |
| Human PMEPA1: | |
| Mouse N4WBP4: | 249 NKEKEKQKGHPL 260 SEQ ID NO. 3 |

The WW domains of NEDD4 protein facilitate its binding to the target proteins via interaction with the PY motifs of NEDD4 binding proteins [Jolliffe et al., Biochem. J., 351: 557-565, 2000; Sudol M, Trends Biochem. Sci., 21: 161-163, 1996; Harvey et al., J. Biol. Chem., 277: 9307-9317, 2002; Macias et al., Nature, 382: 646-649, 1996; Chen et al., Proc. Natl. Acad. Sci., U S A., 92: 7819-7823, 1995; and Murillas et al., J. Biol. Chem., 277: 2897-2907, 2002]. The PMEPA1 protein sequence comprises two PY motifs, i.e., PPPY (SEQ ID NO:15) ("PY1") and PPTY (SEQ ID NO:16) ("PY2"). PY1 is in the central region of the PMEPA1 protein and PY2 is close to the carboxyl terminus of the PMEPA1 protein (Table 2). Therefore, the high protein sequence identity of PMEPA1 with N4WBP4 and the presence of PY motifs indicates that PMEPA1 is the human homolog of *N4WBP4* and can bind to the NEDD4 protein and other proteins containing a WW domain.

### EXAMPLE 4

### PMEPA1-PY Motifs Interact with the WW Domains of NEDD4

**Plasmids.** Mammalian expression vectors encoding PMEPA1-V5 and PMEPA1-GFP fusion proteins were generated by PCR amplification of the PMEPA1 open reading frame. For PMEPA1-V5-pcDNA3.1 vector the following primers were used:

5'-GCTGCTGGAGAACTGAAGGCG-3' (SEQ ID NO:4) and

5'-GTGTCCTTTCTGTTTATCCTTC-3' (SEQ ID NO:5).

For PMEPA1-GFP-pEGFP-vector the primers used were:

5'-AAGCTTGCTGCTGGAGAACTGAAGG CG-3' (SEQ ID NO:6) and

5'-GAATTCGGTGTCCTTTCTGTTTATC-3' (SEQ ID NO:7).

The V5 tag or GFP protein was fused at the carboxyl terminus of the PMEPA1 protein. The PCR product for generating PMEPA1-V5 was inserted into pcDNA3.1-V5-His expression vector (Invitrogen, Carlsbad, CA). The PCR product for generating PMEPA1-GFP was digested by HindIII and EcoRI and cloned into the same sites of pEGFP vector (Clontech, Palo Alto, CA). PMEPA1-PY motif mutants, in which the tyrosine residue (Y) was replaced with an alanine residue (A), were created by using QuikChange Site-Directed Mutagenesis kit (Stratagene, La Jolla, CA) and using the PMEPA1-V5-pcDNA3.1 vector as a template. The plasmids of PMEPA1-*PY* motif mutants are as follows: PMEPA1-*PY1m*-V5-pcDNA3.1, with the first PY motif mutation (Y126A), PMEPA1-*PY2m*-V5-pcDNA3.1, with the second PY motif mutation (Y197A), and PMEPA1-*PY1m*/*PY2m*-V5-pcDNA3.1, with both the PY motif mutations (Y126A and Y197A). The sequences of all the inserts in expression vectors were verified by DNA sequencing.

A bacterial expression plasmid of human *NEDD4* gene (p*NEDD4WW*-GST- pGEX-2TK) encoding all four WW-domains (Accession number XM_046129) fused to glutathione S-transferase (GST-WW fusion protein), was generated by PCR amplification of the coding region of the four WW-domains using the primers:

5'-GCAGGATCCCAACCAGATGCTGCTTGC-3' (SEQ ID NO:8) and

5'-GCAGAATTCTTTTGTAATCCCTGGAGTA-3'(SEQ ID NO:9). Normal prostate tissue derived cDNA was used as a PCR template and the amplified fragment was cloned into the BamHI/EcoRI sites of pGEX-2TK (Amersham Biotech, Piscataway, NJ). A mammalian expression vector (*NEDD4*-GFP-pEGFP) encoding NEDD4-GFP fusion protein was generated using the following primers to generate the *NEDD4* gene fragment by PCR.:

5'-GCAAAGCTTGTCCGGTTTGCTGGAAGC-3' (SEQ ID NO:10) and

5'-GCAGAATTCCCTTTTTGTTCTTATTGGTGAC-3' (SEQ ID NO:11).

**PMEPA1 and NEDD4 Protein Binding Assays.** The *in vitro* binding of PMEPA1 and NEDD4 was assessed by GST pull-down assays. GST-WW fusion protein was prepared and purified with glutathione-Sepharose beads per Amersham Biotech instructions. [³⁵S]methionine labeled proteins representing PMEPA1 and its mutants were generated by *in vitro* transcription/translation (TNT T7 quick coupled transcription/translation system, Promega, Madison, WI). Briefly, the PMEPA1-V5-pcDNA3.1 or the three mutants (2 µg) were incubated in 40 µl of reticulocyte lysate with 40 µCi of [³⁵S]methionine for 1.5 hrs at 30°C.

[³⁵S]methionine incorporation into protein was measured and samples were equalized on the basis of cpm. The GST-WW fusion protein bound to glutathione-Sepharose beads (5 µg) was incubated with the [³⁵S]methionine labeled lysates (12 µl) in 0.4 ml of phosphate-buffered saline (PBS, pH 7.4), 1 mM dithiothreitol, and protease inhibitors. The negative control for each [³⁵S]methionine labeled lysate represented a reaction mixture with equivalent amount of the lysate incubated with glutathione-Sepharose beads without GST-WW fusion protein. After 16 hours of incubation at 4°C, the beads were washed six times with PBS, resuspended in SDS-PAGE sample buffer and run on 12% SDS-PAGE gel under a reducing condition. The gels were dried and autoradiographed.

**Results.** The interaction of PMEPA1 and NEDD4 proteins in cells was evaluated by a co-immunoprecipitation assay. 293 cells (human embryonal kidney cells) were co-transfected with *NEDD4*-GFP-pEGFP vector and one of the PMEPA1-V5 expression vectors encoding either wt PMEPA1-V5 or the PY mutants of PMEPA1. Thirty-six hours later the cells were collected and lysed and the lysates were immunoprecipitated with anti-GFP antibody (Clontech, Palo Alto, CA) following the manufacturer's protocol. The immunoprecipitated proteins were subjected to immunoblotting with an anti-V5 tag antibody (Invitrogen).

*In vitro* translated [³⁵S]Methionine-labeled PMEPA1-V5 fusion protein, with the two intact PY motifs, showed binding to the GST-WW fusion protein (Fig. 6, lane 1). PMEPA1 with PY1 or PY2 mutations revealed significantly decreased binding to WW domains (Fig. 6, lane 2 and lane 3). Further, PMEPA1-V5 and NEDD4-GFP fusion proteins expressed in 293 cells showed strong association (Fig. 7, lane 1) and the mutant PMEPA1-V5 proteins having single mutation of PY1 or PY2 motif or double mutations of both PY1 and PY2 motifs exhibited significantly reduced binding to NEDD4 (Fig. 7, lanes 2, 3, and 4). Thus both *in vitro* and cell culture data reveal that PMEPA1 interacts with NEDD4 and this interaction involves the binding of the PMEPA1 PY motifs to WW domains. The PY2 motif mutation appeared to have a greater effect on binding of PMEPA1 to the NEDD4 WW domain.

The high protein sequence identity of PMEPA1 with N4WBP4 suggests that PMEPA1 is the human homolog of *N4WBP4.*

### EXAMPLE 5

### PMEPA1 Down Regulates Androgen Receptor and Affects Transcriptional Targets of the Androgen Receptor

Example 5 is included for comparative purposes only and does not fall within the scope of the invention.

LNCaP cells were stably transfected with PMEPA1-*GFP* (*PMEPA-GFP*-LNCaP) and pEGFP control (pEGFP-LNCaP) expression vectors. To evaluate the effects of exogenous PMEPA1 expression on androgen receptor in LNCaP transfectants, cells were maintained in androgen-free media for 5 days which is known to down regulate endogenous PMEPA1 expression. Androgen receptor expression was evaluated in these cells after 5 days in the androgen free media (time, 0hr). Androgen receptor expression was also evaluated in cells replenished with 0.1 nM R1881 for different time points (12 hours and 24 hours) after androgen withdrawal. Western blot analysis revealed reduced expression of androgen receptor protein in *PMEPA-GFP*-LNCaP cells (Fig. 4A). Decreased androgen receptor protein levels in PMEPA1 transfectants correlated with the reduced levels of PSA protein, a likely consequence of the attenuation of PSA gene expression due to relatively low levels of androgen receptor protein. PMEPA1 down-regulation of androgen receptor was further supported by results of relative increase of PSMA levels whose expression is normally down regulated by androgen receptor. These experiments showed that PMEPA1 down regulated androgen receptor, and androgen receptor transcriptional targets were affected correspondingly.

Because PMEPA1 is a *NEDD4* binding protein, its effects on androgen receptor expression may involve the ubiquitin-proteasome pathway. To show that PMEPA1's effect on androgen receptor expression does not result from a general or non-specific effect of the upregulation of a ubiquitin protein ligase in the protein degradation pathway, we evaluated the effects of PMEPA1 on androgen receptor and the p27 protein, which is known to be degraded through a ubiquitin-dependent pathway. We generated a stable PMEPA1-GFP-Tet-LNCaP transfectant, in which the expression of PMEPA1-GFP fusion protein is regulated by tetracycline (Tet-off system, Clontech). As shown in Figure 4B, cells cultured in the medium with tetracycline lacked PMEPA1 expression (Tet-off) but overexpressed PMEPA1 when cultured in the medium without tetracycline. The protein level of androgen receptor decreased dramatically in PMEPA1-overexpressing cells as compared to the relative expression of p27 or tubulin (Fig. 4B). Taken together, these data show that androgen receptor is a specific target of PMEPA1.

### EXAMPLE 6

### Golgi Association of PMEPA1 Protein.

Our studies also revealed that PMEPA1 is a Golgi-associated protein.

**Immunofluorescence Assays**. Plasmids were prepared as discussed above in Example 4. The immunofluorescent assays were performed following the procedure described by Harvey et al., J. Biol. Chem., 277: 9307-9317, 2002. Briefly, stable transfectants of LNCaP cells harboring PMEPA1-GFP-pEGFP (LNCaP-PMEPA1-GFP transfectant) were grown on coverslips for two days, fixed in 2% paraformaldehyde for 15 minutes and permeabilized in 0.2% Triton X-100 for 2 minutes. Fixed and permeabilized cells were incubated with anti-GM130 (recognizes a cis-Golgi matrix protein) or anti-TGN38 (recognizes a protein localizing to Trans-Golgi Network, TGN) monoclonal antibodies (BD Transduction Laboratory, San Diego, CA) at 6.25 µg/ml for 30 minutes at room temperature. Cells were then washed to remove excess or non-specifically bound primary antibody followed by incubation with TRITC conjugated anti-mouse antibody (Sigma, ST. Louis, MO) at 1:100 dilution for 30 minutes at room temperature. The sections were mounted with fluoromount (Southern Associates, Birmingham, AL) and the images were processed with a Leica fluoromicroscope and Open-Lab software (Improvision, Lexington, MA).

**Results.** PMEPA1-GFP fusion protein showed peri-nuclear localization with a Golgi-like appearance. The images of sub-cellular location of GM130, a cis-Golgi protein, showed similar pattern as PMEPA1-GFP fusion protein. Superimposing the images of PMEPA1-GFP fusion protein and GM130 in LNCaP-PMEPA1-GFP transfectants confirmed the localization of PMEPA1-GFP fusion protein on cis-Golgi structure. We did not observe the co-localization of PMEPA1-GFP and TGN-38, which localizes to TGN.

The sub-cellular localization of PMEPA1 is similar to two other newly identified NEDD4 WW domain binding proteins, N4WBP5 and N4WBP5a, which also localize to the Golgi complex [Harvey et al., J. Biol. Chem., 277: 9307-9317, 2002; Konstas et al., J. Biol. Chem., 277: 29406-29416, 2002]. N4WBP5a sequestered the trafficking of NEDD4/NEDD4-2 thereby increasing the activity of the epithelial sodium channel (EnaC), a known target down regulated by *NEDD4* [Konstas et al., J. Biol. Chem., 277: 29406-29416, 2002]. As a highly androgen-regulated gene and a NEDD4 binding protein, the localization of PMEPA1 on the Golgi apparatus suggests that PMEPA1 is involved in protein turn-over of androgen receptor targets.

### EXAMPLE 7

### PMEPA1 Inhibits Growth of Prostate Cancer Cells.

**Colony-Forming Assays.** To investigate the biologic effects of PMEPA1 expression in regulating cell growth and the contribution of PY motifs to such functions, we performed the colony-formation assay by transfecting various prostate cancer cell lines with expression vectors of the wild type PMEPA1 ("wt-PMEPA1") and PMEPA1-*PY* mutants.

Prostate cancer cell lines: LNCaP, PC3, and DU145 were purchased from ATCC (Rockville, MD) and grown in the cell culture media as described by the supplier. The LNCaP sub-lines C4, C4-2 and C4-2B [Hsieh et al., Cancer Res., 53: 2852-7, 1993; Thalmannet al., Cancer Res., 54: 2577-81, 1994; and Wu et al., Int. J. Cancer, 77: 887-94, 1998] were purchased from Urocor (Oklahoma, OK) and cultured in T medium (5% FBS, 80% DMEM, 20% F12, 5ug/ml insulin, 13.65pg/ml Triiodo-Thyronine, 5ug/ml apo-transferrin, 0.244ug/ml biotin, 25ug/ml adenine).

Three micrograms of plasmids (PMEPA1-V5-pcDNA3.1 or vector without PMEPA1 insert) were transfected into the 50-70% confluent cells in triplicate in 60-mm petri dishes with Lipofectamine (Invitrogen, Carlsbad, CA). Tumor suppressor gene p53 (wt), and mt p53 (R175H and G245D) were also used in parallel as controls. Approximately 36 hours later, selection with G418 at 800 µg/ml (DU145 and PC3) or 400 µg/ml (LNCaP and its sublines) was initiated. Cells were maintained with G418-containing medium that was changed every 3-4 days. After 2-4 weeks of selection, the cells were rinsed with 1x PBS, fixed with 2% formaldehyde in 1xPBS for 15 minutes, stained with 0.5% crystal violet in 1xPBS for 15 minutes, and rinsed 1-2 times with distilled H₂O. Colonies visible in each dish without magnification were counted by Open-Lab software.

To assess the effects of the PY motif mutations on the colony-forming ability of PMEPA1, LNCaP and PC3 cells were also transfected with PMEPA1 mutants: PMEPA1-*PY1m*-pcDNA3.1, PMEPA1-*PY2m*-pcDNA3.1, or PMEPA1-*PY1m*/*PY2m*-pcDNA3.1. PMEPA1-V5-pcDNA3.1 and expression vector without insert served as positive and negative controls, respectively, for the PMEPA1 mutants. Two independent colony-forming assays were performed as above.

As shown in Fig. 3A-F, the colony-forming abilities of prostate cancer cell lines DU145, PC3, LNCaP, and LNCaP sublines were significantly suppressed by transfection of the sense version of the wt-PMEPA1 expression vector. Under these conditions wt-p53 showed similar cell growth inhibition (data not shown).

In two independent experiments, mutation of the PY1 motif appears to abolish the inhibition of colony formation by wt-PMEPA1, emphasizing the role of the PY1 motif in PMEPA1 and NEDD4 interactions and the biologic functions of PMEPA1 (Fig. 3G-H). The growth inhibitory effect of PMEPA1 appears to be linked to the interactions of PY1 motif to NEDD4 WW domain. This interpretation is based on the striking observations showing distinctively more colonies with PY1 motif mutant in comparison to wt-PMEPA1.

Cell Proliferation Analysis. To further evaluate the growth inhibitory effects of PMEPA1 on prostate cancer cells, a stable PMEPA1-GFP-Tet LNCaP transfectant was generated. Expression of PMEPA1-GFP fusion protein in these cells was negatively regulated by tetracycline in the medium (Clontech). For cell proliferation assays, three thousand PMEPA1-GFP-Tet LNCaP cells were seeded in 96-well plates with or without 1 µg/ml of tetracycline in the medium. CellTiter 96 Aqueous One Solution kit (Promega, Madison, WI) was used to measure the cell proliferation according to the manufacturer's instructions.

The growth inhibitory effect of PMEPA1 has been further confirmed by the cell proliferation characteristics of stable PMEPA1-GFP-Tet-LNCaP cells, where exogenous PMEPA1 is upregulated in the absence of tetracycline. The growth of the PMEPA1-GFP-Tet LNCaP cells in tetracycline negative medium is significantly slower than that of PMEPA1-tet LNCaP transfectant in tetracycline positive medium (Fig. 5). LNCaP cells with PMEPA1 overexpression also revealed increased RB phosphorylation further confirming the cell growth inhibitory effect of PMEPA1 (data not shown).

PMEPA1 is expressed in androgen receptor positive prostate cancer cell lines, including LNCaP and its sublines (C4, C4-2 and C4-2B). LNCaP cells are androgen dependent for growth. Even though the growth of LNCaP sublines is androgen independent, androgen receptor is critical for their proliferation [Zegarra-Moro et al., Cancer Res., 62: 1008-1013, 2002]. We observed that overexpression of PMEPA1 by transfecting the PMEPA1 expression vector into LNCaP and its sublines significantly inhibited the cell proliferation. Since our preliminary observations showed that PMEPA1 overexpression in LNCaP cells resulted in altered expression of androgen receptor downstream genes (Xu et al. unpublished data), we hypothesized that the growth inhibitory effect of PMEPA1 on LNCaP and its sublines may be mediated directly or indirectly through affecting androgen receptor functions. Despite the growth inhibitory effect on androgen receptor positive prostate cancer cell lines, PMEPA1 was also found to inhibit the growth of androgen receptor negative prostate tumor cells, DU145 and PC3, suggesting that the growth inhibitory effects of PMEPA1 on DU145 and PC3 could be mediated through alternative mechanisms, e.g., regulation of other nuclear steroid receptors by PMEPA1. Nonetheless, inhibition of prostate cancer cell growth by PMEPA1 implicates PMEPA1 in control of prostate cancer , development.

### EXAMPLE 8

### Decreased PMEPA1 Expression in Prostate Tumor Tissues.

We also evaluated the relationship of alterations in PMEPA1 expression to the clinico-pathologic features of prostate cancer.

### Prostate Tissue Specimens, Laser Capture Microdissection (LCM) and Quantitative RT-PCR (QRT-PCR) Assay.

Matched prostate cancer and normal tissues were derived from radical prostatectomy specimens from 62 CaP patients treated at Walter Reed Army Medical Center (under an IRB-approved protocol). The procedures of collecting specimens were previously described [Xu et al., Cancer Res. 60: 6568-6572, 2000]. Ten micron frozen sections were prepared and stored at - 70°C. Histologically normal prostate epithelial cells and prostate tumor cells from each patient were harvested using LCM equipment according to the protocol provided by the manufacturer (Arcturus Engineering, Mountain View, CA).

Total RNA was prepared from the harvested normal and tumor prostate epithelial cells as previously described [Xu et al., Cancer Res. 60: 6568-6572, 2000] and quantified with Fluorometer (Bio-Rad, Hercules, CA). QRT-PCR was conducted using 0.1 ng of total RNA from paired normal and tumor cells. PMEPA1 PCR primers were carefully designed that only amplify PMEPA1 but not STAG1, an alternatively spliced form of PMEPA1 [Rae et al., Mol. Carcinog., 32: 44-53, 2001]. The PCR primers were:

5'-CATGATCCCCGAGCTGCT-3' (SEQ ID NO:12) and

5'-TGATCTGAACAAACTCCAGCTCC-3' (SEQ ID NO:13), and the labeled probe was:

5'-AGGCGGACAGTCTCCTGCGAAAC-3' (SEQ ID NO:14).

GAPDH gene expression was detected as the internal control (PE Applied Biosystems, Foster, CA). Paired triplicate samples (one lacking RT and duplicate with RT) were amplified in 50 µl volumes containing the manufacturer's recommended universal reagent, proper primers and probe of PMEPA1 or GAPDH using 7700 sequence detection system (PE Applied Biosystems, Foster, CA).

Results were plotted as average cycle threshold (cT) values for each duplicate sample minus the average duplicate cT values for GAPDH. Differences between matched tumor (T) and normal (N) samples were calculated using 2exp(cTₜᵤₘₒᵣ-cTₙₒᵣₘₐₗ) and expressed as fold changes in expression. The expression status of PMEPA1 was further categorized as either: 1) overexpression in tumor tissue (T>N), defined as 1+ (1.5-3 fold), 2+ (3.1-10 fold), 3+ (10.1-20 fold) and 4+ (>20 fold) increased expression as compared with matched normal tissue; 2) reduced expression in tumor tissue (T<N), defined as 1- (1.5-3 fold), 2- (3.1-10 fold), 3- (10.1-20 fold) and 4- (>20 fold) decreased expression as compared with matched normal tissue; or 3) no change (T=N), defined as 0 (< 1.5 fold). No detectable PMEPA1 expression in one of the specimens of tumor/normal pairs was scored as 4+ for increased or 4- for decreased expression.

Statistical analysis was performed with the SPSS software package. The association between PMEPA1 expression and clinico-pathological features was analyzed using chi-square tests. The Kaplan-Meier curves were applied to display the PSA-recurrence-free survival data. A p value < 0.05 was considered as statistically significant.

The overall expression pattern of PMEPA1 primary prostate cancer is shown below in Table 3.

**Table 3**

| PMEPA1 Expression | Number of Patients/ Group (%) | Degree of PMEPA1 Expression | |
|---|---|---|---|
| | | Quantity | Number (%) |
| T<N | 40 (64.5) | 1- | 11 (27.5) |
| | | 2- | 17 (42.5) |
| | | 3- | 5 (12.5) |
| | | 4- | 7 (17.5) |
| T>N | 10 (16.1) | 1+ | 6 (60.0) |
| | | 2+ | 4 (40.0) |
| | | 3+ | |
| | | 4+ | |
| T=N | 12 (19.4) | 0 | |

Comparison of PMEPA1 expression between tumor and normal cells revealed tumor cell associated decreased expression (T<N) in 64.5% tumor specimens (40 of 62), increased expression (T>N) in 16.1% specimens (10 of 62) and no change (T=N) in 19.4% specimens (12 of 62). When these expression patterns were stratified by organ-confined (pT2) and non-organ-confined (pT3) disease, a higher percentage of PMEPA1 reduction was seen in pT3 (74%) vs. pT2 (48%). Because the T>N group has a small number of cases, we combined the T>N group and the T=N group (T ≥ N group). As shown below in Table 4, comparison of the clinico-pathologic parameters between the T<N group and the T ≥ N group revealed that the T<N group had a significantly higher percentage of patients with pT3 tumors (p = 0.035) and more patients in this group had a higher level of preoperative serum prostate specific antigen (PSA) (p = 0.023).

**TABLE 4**

| **PMEPA1 Expression** | **Pathologic Stage (%)** | | **PSA Range (%)** | | | **PSA Recurrence (%)** | | **Time to Recurrence after Surgery (month)** |
|---|---|---|---|---|---|---|---|---|
| | T2 | T3 | ≤ 4 ng/ml | 4.1-10 ng/ml | 10.1-20 ng/ml | No | Yes | Mean ± SE |
| **T<N** | 11 (27.5) | 29 (72.5) | 1 (2.5) | 30 (75.0) | 9 (22.5) | 29 (72.5) | 11 (27.5) | 8.2 ± 3.4 |
| **T≥N** | 12 (54.5) | 10 (45.5) | 5 (22.7) | 15 (68.2) | 2 (9.1) | 19 (86.4) | 3 (13.6) | 18.4 ± 6.3 |
| **p Value** | 0.035 | | 0.023 | | | 0.211 | | 0.18 |

Out of 62 patients whose tumors were analyzed for PMEPA1 expression, 14 patients showed prostate cancer recurrence as defined by serum PSA level equal or higher than 0.2 ng/ml after prostatectomy. Of the 14 patients, 11 showed reduced tumor associated PMEPA1 expression (78.5%). Reduced PMEPA1 expression seems to associate with a higher recurrence rate and a shorter duration to recurrence after surgery, even through the statistical analysis did not reveal a significant difference. The absence of a significant difference might be due to the small number of patients.

## Claims

1. Use of a polypeptide comprising SEQ ID NO:2 in the manufacture of a medicament for inhibiting the growth of a prostate cancer cell.

2. Use of a vector that comprises a polynucleotide that encodes a polypeptide having the sequence of SEQ ID NO:2 in the manufacture of a medicament for inhibiting the growth of a prostate cancer cell.

3. The use according to claim 2, wherein the polynucleotide comprises SEQ ID NO:1.

4. A polypeptide variant of SEQ ID NO:2, wherein said variant consists of SEQ. ID. No. 2 having one or more mutations and/or deletions in one or both of the PY motifs of SEQ. ID. No. 2; wherein said PY motifs consist of SEQ. ID. Nos. 15 and 16.

5. A method for diagnosing prostate cancer comprising:
a) detecting the expression levels of a polypeptide or a polynucleotide in a biological sample, wherein the polypeptide comprises the amino acid sequence of SEQ ID NO:2 and the polynucleotide encodes a protein having the amino acid sequence of SEQ ID NO:2, and
b) determining a probability of prostate cancer development, wherein reduced expression of the polynucleotide or the polypeptide in the biological sample correlates with an increased probability of cancer development.

6. The method of claim 5, wherein the polynucleotide comprises SEQ ID NO:1.

7. The method according to claim 5, wherein the expression of the polynucleotide is reduced as compared to a control sample.

8. A method of prognosing prostate cancer comprising:
a) detecting the expression levels of a polypeptide or a polynucleotide in a biological sample, wherein the polypeptide comprises an amino acid sequence that is at least 95% identical to SEQ ID NO:2 and the polynucleotide encodes a protein that is at least 95% identical to SEQ ID NO:2; and
b) determining a probability of prostate cancer progression, wherein reduced expression of the polypeptide or the polynucleotide in the biological sample correlates with an increased probability of non-organ confined prostate cancer or an increased recurrence rate of prostate cancer following prostate surgery.

9. The method according to claim 8, wherein the polypeptide is detected with an antibody that specifically binds to the polypeptide.

10. The method according to claim 8, wherein the expression of the polypeptide or the polynucleotide is reduced as compared to a control sample.

11. The method according to claim 8, wherein the biological sample is obtained from a patient who has undergone prostate cancer therapy.

12. The method according to claim 11, wherein the therapy is prostate surgery.

13. The method according to claim 8, wherein the polypeptide comprises SEQ ID NO:2

14. Use of a polypeptide comprising an amino acid sequence that is at least 95% identical to SEQ ID NO:2 in the manufacture of a medicament for inhibiting the growth of a prostate cancer cell.

## Patentansprüche

1. Verwendung eines SEQ ID NO:2 umfassenden Polypeptids bei der Herstellung eines Medikaments zum Inhibieren des Wachstums einer Prostatakrebszelle.

2. Verwendung eines Vektors, der ein Polynukleotid umfasst, das für ein Peptid codiert, das die Sequenz SEQ ID NO:2 aufweist, bei der Herstellung eines Medikaments zum Inhibieren des Wachstums einer Prostatakrebszelle.

3. Verwendung nach Anspruch 2, worin das Polynukleotid SEQ ID NO:1 umfasst.

4. Polypeptidvariante von SEQ ID NO:2, worin die Variante aus SEQ ID NO:2 mit einer oder mehreren Mutationen und/oder Deletionen in einem oder beiden der PY-Motive von SEQ ID NO:2 besteht; worin die PY-Motive aus den SEQ ID NO:15 und 16 bestehen.

5. Verfahren zum Diagnostizieren von Prostatakrebs, umfassend:
a) Nachweisen der Expressionsgrade eines Polypeptids oder eines Polynukleotids in einer biologischen Probe, worin das Polypeptid die Aminosäuresequenz von SEQ ID NO:2 umfasst und das Polynukleotid für ein Protein codiert, das die Aminosäuresequenz von SEQ ID NO:2 aufweist; und
b) Bestimmen einer Wahrscheinlichkeit einer Prostatakrebsentwicklung, worin verringerte Expression des Polynukleotids oder des Polypeptids in der biologischen Probe mit einer erhöhten Wahrscheinlichkeit der Entwicklung von Krebs korreliert.

6. Verfahren nach Anspruch 5, worin das Polynukleotid SEQ ID NO:1 umfasst.

7. Verfahren nach Anspruch 5, worin die Expression des Polynukleotids im Vergleich mit einer Vergleichsprobe verringert ist.

8. Verfahren zum Prognostizieren von Prostatakrebs, umfassend:
a) Nachweisen der Expressionsgrade eines Polypeptids oder eines Polynukleotids in einer biologischen Probe, worin das Polypeptid eine Aminosäuresequenz umfasst, die zu mindestens 95% identisch ist mit SEQ ID NO:2, und worin das Polynukleotid für ein Protein codiert, das zu mindestens 95% identisch ist mit SEQ ID NO:2; und
b) Bestimmen einer Wahrscheinlichkeit der Prostatakrebsprogression, worin verringerte Expression des Polypeptids oder des Polynukleotids in der biologischen Probe mit einer erhöhten Wahrscheinlichkeit eines Nicht-Organ-begrenzten Prostatakrebses oder einer erhöhten Wiederauftrittsrate von Prostatakrebs nachfolgend auf Prostataoperation korreliert.

9. Verfahren nach Anspruch 8, worin das Polypeptid mit einem Antikörper nachgewiesen wird, der spezifisch an das Polypeptid bindet.

10. Verfahren nach Anspruch 8, worin die Expression des Polypeptids oder des Polynukleotids im Vergleich mit einer Kontrollprobe verringert ist.

11. Verfahren nach Anspruch 8, worin die biologische Probe von einem Patienten erhalten wird, der eine Prostatakrebstherapie eingegangen ist.

12. Verfahren nach Anspruch 11, worin die Therapie Prostataoperation ist.

13. Verfahren nach Anspruch 8, worin das Polypeptid SEQ ID NO:2 umfasst.

14. Verwendung eines Polypeptids, das eine Aminosäuresequenz umfasst, die zu mindestens 95% identisch ist mit SEQ ID NO:2 bei der Herstellung eines Medikaments zum Inhibieren des Wachstums einer Prostatakrebszelle.

## Revendications

1. Utilisation d'un polypeptide comprenant la SEQ ID N° 2 dans la production d'un médicament destiné à inhiber la croissance d'une cellule de cancer de la prostate.

2. Utilisation d'un vecteur qui comprend un polynucléotide qui code pour un polypeptide ayant la séquence de la SEQ ID N° 2 dans la production d'un médicament destiné à inhiber la croissance d'une cellule de cancer de la prostate.

3. Utilisation suivant la revendication 2, dans laquelle le polynucléotide comprend la SEQ ID N° 1.

4. Variant polypeptidique de la SEQ ID N° 2, ledit variant consistant en la SEQ ID N° 2 comportant une ou plusieurs mutations et/ou délétions dans l'un des ou les deux motifs PY de la SEQ ID N° 2, lesdits motifs PY consistant en les SEQ ID N° 15 et 16.

5. Méthode pour le diagnostic du cancer de la prostate, comprenant :
a) la détection des degrés d'expression d'un polypeptide ou d'un polynucléotide dans un échantillon biologique, le polypeptide comprenant la séquence d'aminoacides de la SEQ ID N° 2 et le polynucléotide codant pour une protéine ayant la séquence d'aminoacides de la SEQ ID N° 2 ; et
b) la détermination d'une probabilité de développement du cancer de la prostate, où une expression réduite du polynucléotide ou polypeptide dans l'échantillon biologique présente une corrélation avec une probabilité accrue de développement du cancer.

6. Méthode suivant la revendication 5, dans laquelle le polynucléotide comprend la SEQ ID N° 1.

7. Méthode suivant la revendication 5, dans laquelle l'expression du polynucléotide est réduite, comparativement à un échantillon témoin.

8. Méthode pour le pronostic du cancer de la prostate, comprenant :
a) la détection des degrés d'expression d'un polypeptide ou d'un polynucléotide dans un échantillon biologique, le polypeptide comprenant une séquence d'aminoacides qui est identique à au moins 95 % à la SEQ ID N° 2 et le polynucléotide codant pour une protéine qui est identique à au moins 95 % à la SEQ ID N° 2 ; et
b) la détermination d'une probabilité de progression du cancer de la prostate, une expression réduite du polynucléotide ou polypeptide dans l'échantillon biologique présentant une corrélation avec une probabilité accrue d'un cancer de la prostate non confiné à l'organe ou un taux de récidive accru du cancer de la prostate après une intervention chirurgicale de la prostate.

9. Méthode suivant la revendication 8, dans laquelle le polypeptide est détecté avec un anticorps qui se lie spécifiquement au polypeptide.

10. Méthode suivant la revendication 8, dans laquelle l'expression du polypeptide ou du polynucléotide est réduite, comparativement à un échantillon témoin.

11. Méthode suivant la revendication 8, dans laquelle l'échantillon biologique est obtenu à partir d'un patient qui a subi une thérapie du cancer de la prostate.

12. Méthode suivant la revendication 11, dans laquelle la thérapie est une intervention chirurgicale de la prostate.

13. Méthode suivant la revendication 8, dans laquelle le polypeptide comprend la SEQ ID N° 2.

14. Utilisation d'un polypeptide comprenant une séquence d'aminoacides qui est identique à au moins 95 % à la SEQ ID N° 2 dans la production d'un médicament destiné à inhiber la croissance d'une cellule de cancer de la prostate.
